# EUROPEAN PATENT SPECIFICATION

(11) **EP 3 622 928 B1**
(45) Date of publication and mention of the grant of the patent: **31.03.2021**
(21) Application number: 18813136.1
(22) Date of filing: 19.02.2018
(51) Int. Cl.: A61F 13/496, A61F 13/15

(54) **ABSORBENT ARTICLE**
ABSORBIERENDER ARTIKEL
ARTICLE ABSORBANT

(30) Priority: 05.06.2017 JP 2017110867
(43) Date of publication of application: 18.03.2020
(73) Proprietor: Unicharm Corporation, Ehime 799-0111 (JP)
(72) Inventor: SONODA, Junko, Kanonji-shi Kagawa 769-1602 (JP); ICHIKAWA, Makoto, Kanonji-shi Kagawa 769-1602 (JP); KAWABATA, Kuniyoshi, Kanonji-shi Kagawa 769-1602 (JP); TANAKA, Yoshinori, Kanonji-shi Kagawa 769-1602 (JP)
(74) Representative: Dolleymores
(86) International application number: PCT/JP2018/005799
(87) International publication number: WO 2018/225302

(56) References cited:
- EP-A1- 2 484 323
- EP-A1- 2 526 913
- WO-A1-2015/118735
- JP-A- 2009 501 603
- JP-A- 2016 526 963
- JP-A- 2017 148 111

## Description

### FIELD

The present invention relates to an absorbent article.

### BACKGROUND

Absorbent articles (for example, pants-type disposable diapers) are known that include an abdomen side section and a dorsal side section, with at least one edge section of the abdomen side section and one edge section of the dorsal side section in the widthwise direction overlapping in the thickness direction along the lengthwise direction, and being joined together at least one joint section. Patent Literature 1, for example, discloses a pants-type disposable diaper. The pants-type disposable diaper includes a pair of joint sections (side seal sections), each of the pair of joint sections having a plurality of welded sections formed by heat sealing or ultrasonic sealing. Also, Patent Literature 2 discloses a pants-type disposable diaper. The disposable diaper has ones of both edge sections in the widthwise direction of the abdomen side section and dorsal side section (both right and left side sections of the waist opening) joined together to form joint sections, while the other edge sections are un-joined, forming joining means that can be joined together. Patent Literature 3 discloses an apparatus for carrying out ultrasonic sealing.

### [CITATION LIST]

### [PATENT LITERATURE]

[PTL 1] Japanese Unexamined Patent Publication No. 2013-146419
[PTL 2] Japanese Unexamined Utility Model Publication H 4-5826
[PTL 3] Japanese Unexamined Patent Publication No. 2006-192902

### SUMMARY

### [TECHNICAL PROBLEM]

When a wearer of an absorbent article such as a pants-type disposable diaper, or a caregiver of the wearer, removes the used absorbent article from the wearer, it is common to adopt a method whereby the abdomen side section and dorsal side section are detached at the joint sections along the lengthwise direction. For removal, therefore, it is necessary for the abdomen side section and the dorsal side section to be joined at the joint sections in an easily separable manner along the lengthwise direction. When the absorbent article is being worn by the wearer, however, force is applied to the joint sections mainly in the left-right direction of the wearer, i.e. in the widthwise direction. While it is being worn, therefore, it is necessary for the abdomen side section and the dorsal side section to be joined at the joint sections in a manner so that they are unlikely to separate in the widthwise direction. The same also applies to absorbent articles in which both edge sections of the abdomen side section and dorsal side section in the widthwise direction (both the right and left side sections of the waist opening) are joined together to form a pair of joint sections, and to absorbent articles in which one of the both edge sections are joined together to form a joint section while the other is un-joined to form joining means that can be joined together. It is therefore desirable for separation of the abdomen side section and dorsal side section to be difficult when the absorbent article is being worn, and for separation of the abdomen side section and dorsal side section to be easy when it is to be removed.

In Patent Literature 1, the area of each welded section in the regions near the waist opening is relatively small while the area of each welded section in the regions further from the waist opening is relatively large. In the regions near the waist openings in this case, the abdomen side section and dorsal side section are presumably easier to separate, compared to the regions further from the waist opening. At the welded sections, however, all of the plurality of sheets at the abdomen side section and the plurality of sheets at the dorsal side section are laminated, melted and pressed, thereby being welded together. In order to detach the abdomen side section and dorsal side section, therefore, it is necessary to detach the welded sections where all of the sheets of the abdomen side section and dorsal side section are welded, or their surrounding regions. The force used for detachment, i.e. the force used for separation, is not insignificant, therefore, and it may be difficult for the wearer to detach them.

It is an object of the present invention to provide a further improved absorbent article in which separation between the abdomen side section and dorsal side section is difficult when it is worn, while separation in the lengthwise direction is easy when it is to be removed.

### [SOLUTION TO PROBLEM]

The absorbent article of the present invention is (1) an absorbent article including an abdomen side section and a dorsal side section, at least one edge section of the abdomen side section and one edge section of the dorsal side section in a widthwise direction being joined together by a joint section while overlapping in a thickness direction along a lengthwise direction, wherein the joint section includes a plurality of fused joint sections, each of the plurality of fused joint sections includes a fused section where a plurality of sheets on the abdomen side section and a plurality of sheets on the dorsal side section are fused together in the thickness direction, and a side wall section where the plurality of sheets on the abdomen side section and the plurality of sheets on the dorsal side section are fused together in the thickness direction in a manner extending from a perimeter edge of the fused sections, cylindrically in the thickness direction, a location of the fused section in the thickness direction deviates from a borders between the abdomen side section and the dorsal side section, a dimension of the side wall section in the thickness direction is larger than a dimension of the fused section in the thickness direction, and a dimension of the side wall section in the lengthwise direction is smaller than a dimension in the widthwise direction.

The absorbent article includes, at the fused joint section among the joint section, the fused section and side wall section extending from the perimeter edge of the fused section in a cylindrical manner in the thickness direction, the dimension of the side wall section in the lengthwise direction (the cylindrical thickness in the lengthwise direction) being smaller than the dimension in the widthwise direction (the cylindrical thickness in the widthwise direction). The section of the cylinder of the side wall section in the lengthwise direction is therefore easy to tear, while the section in the widthwise direction is difficult to tear. In addition, the dimension of the side wall section in the thickness direction (their thickness) is larger than the dimension of the fused section in the thickness direction (their thickness). In other words, in the fused section, the plurality of sheets on the abdomen side section and the plurality of sheets on the dorsal side section that are layered are joined by fusion while being pressed, for example, such that the dimension in the thickness direction is smaller (thinner). At the fused section, therefore, the plurality of sheets are joined together relatively firmly. At the side wall section, on the other hand, the plurality of sheets on the abdomen side section and the plurality of sheets on the dorsal side section that are layered are joined at the perimeter edge of the fused sections by fusion without being pressed, and therefore the dimension in the thickness direction is larger (thicker). At the side wall section, therefore, the plurality of sheets are joined together relatively weakly. In addition, the location of the fused section in the thickness direction deviates from the border between the abdomen side section and dorsal side section. Therefore when the abdomen side section and dorsal side section are separated, the fused sections are not included within the separated points.

Consequently, when the abdomen side section and dorsal side section of the joint section are detached along the lengthwise direction, tearing can be initiated at the side wall section that is relatively weakly joined, and specifically at the section in the lengthwise direction of the side wall section that is easily torn. This allows the abdomen side section and dorsal side section to be easily detached along their interface, while keeping the fused section that is strongly joined, or its surrounding region, from being detached. In this absorbent article, therefore, it is possible to detach the abdomen side section and dorsal side section of the joint section with relatively small force. Specifically, it is possible to easily detach the abdomen side section and dorsal side section along their interface with a relatively small force, at the section on one side of the side wall section in the lengthwise direction, until from the outer side surface of the cylinder up to the inner side surface of the cylinder of the side wall section. Also, if the abdomen side section and dorsal side section can be detached at the section on one side of the side wall section in the lengthwise direction from the outer side surface of the cylinder to the inner side surface of the cylinder, then it is possible to easily detach the abdomen side section and dorsal side section at the section on the other side in the lengthwise direction, from the inner side surface of the cylinder to the outer side surface of the cylinders of the side wall section. This will allow the abdomen side section and dorsal side section of the joint sections to be easily detached. That is, it will aid the wearer in detaching the abdomen side section and dorsal side section.

When the wearer is wearing the absorbent article, on the other hand, the force applied to the joint section in response to movement of the wearer mainly moves in the widthwise direction. Moreover, in this absorbent article, the dimension of the side wall section in the widthwise direction (the thickness of the cylinder in the widthwise direction) is larger than the dimension in the lengthwise direction (the thickness of the cylinder in the lengthwise direction). When large force has been applied to the side wall section in the widthwise direction, the high-thickness portions of the side wall section in the widthwise direction absorbs the force. Therefore, even if cracks have formed from the outer side surface of the cylinder to the inner side surface of the cylinder of the side wall section, the cracks either go through the upper surface or lower surface of the side wall section, or stop upon reaching the fused section that has high joining strength, before reaching the inner side surface of the cylinder of the side wall section. When it is worn, therefore, detachment of the abdomen side section and dorsal side section of the joint section, and therefore tearing of the absorbent article, can be minimized.

By thus forming side wall sections having short dimensions in the lengthwise direction and long dimensions in the widthwise direction around the fused sections at the fused joint sections, it is possible to make the ease of detachment, i.e. the ease of separation, different in the lengthwise direction and the widthwise direction, or in other words, to provide anisotropy. As a result, the absorbent article is further improved such that it is easy to separate at the time of removal while having the abdomen side section and dorsal side section difficult to separate when it is worn.

The absorbent article of the present invention may also be (2) the absorbent article according to (1) above, wherein the joint section has a pressure welded section row in which the plurality of fused joint sections are arranged along the lengthwise direction with intervals between them.

In this absorbent article, the plurality of fused joint sections are arranged along the lengthwise direction with intervals between them (pressure welded section rows). Therefore when the absorbent article is to be removed, the abdomen side section and dorsal side section can be detached with relatively small force for a relatively long distance along the lengthwise direction. This allows the abdomen side section and dorsal side section of the joint sections to be more easily detached.

The absorbent article of the present invention may also be (3) the absorbent article according to (2) above, wherein the joint section has only a single pressure welded section row as the plurality of fused joint sections.

When it has a plurality of pressure welded section rows in the widthwise direction, the pulling force between the abdomen side section and dorsal side section along the lengthwise direction when the absorbent article is removed is diffused at each row, and cracks produced by detachment run along the diagonal direction with respect to the lengthwise direction or along the widthwise direction, potentially increasing the force required for detachment. It is desirable for the plurality of rows to have the same joining strength, but since the joining strength differs depending on the production yield, a condition may be created in which the fused joint sections in one row do not tear even if the fused joint sections in another row are torn. This absorbent article therefore has only a single pressure welded section row as the plurality of fused joint sections. When the absorbent article is removed, therefore, pulling force between the abdomen side section and dorsal side section along the lengthwise direction can be concentrated to the single pressure welded section row. This allows the abdomen side section and dorsal side section of the joint sections to be more easily detached with a smaller degree of force.

The absorbent article of the present invention may also be (4) the absorbent article according to (2) above, wherein the joint section has, in the lengthwise direction, a waist side edge region including an edge section close to a waist opening, a leg side edge region including an edge section far from the waist opening, and a center region between the waist side edge region and the leg side edge region, and at least the waist side edge region and center region have only a single pressure welded section row as the plurality of fused joint sections.

At the joint section, this absorbent article has a plurality of fused joint sections arranged in a single row in at least the waist side edge region and center region. When the absorbent article is removed, therefore, pulling force between the abdomen side section and dorsal side section along the lengthwise direction can be concentrated at the single row of the plurality of fused joint sections, so that it can reach at least up to the center region in the lengthwise direction of the joint section. This allows the abdomen side section and dorsal side section of the joint section to be easily detached by a smaller degree of force, and can minimize tearing of the joint section in the widthwise direction or tearing of the abdomen side section or dorsal side section in the widthwise direction.

The absorbent article of the present invention may also be (5) the absorbent article according to any one of (1) to (4) above, wherein a maximum dimension in the lengthwise direction of the fused section is greater than or equal to a maximum dimension in the widthwise direction.

In this absorbent article, the maximum dimension in the lengthwise direction of each fused section is greater than the maximum dimension in the widthwise direction, or in other words, the fused sections have long shapes in the lengthwise direction. Thus, the thickness of the cylinder formed by the side wall section can be thinner in the lengthwise direction and thicker in the widthwise direction. When the absorbent article is removed, therefore, the abdomen side section and dorsal side section can be more easily separated from the thin sections, while even large force in the widthwise direction can be stopped by the thick sections of the side wall sections, when the article is being worn, and it is thus possible to help prevent detachment of the abdomen side section and dorsal side section of the joint section, and tearing of the absorbent article as a result.

The absorbent article of the present invention may also be (6) the absorbent article according to any one of (1) to (5) above, wherein a dimension in the lengthwise direction (one side) of the side wall section is 50 µm to 200 µm, and a dimension in the widthwise direction (other side) of the side wall section is 100 µm to 400 µm.

In this absorbent article, the dimension in the lengthwise direction of the side wall section is 50 µm to 200 µm while the dimension in the widthwise direction of the side wall sections is 100 µm to 400 µm.Consequently, the abdomen side section and dorsal side section can be easily detached when a large force is applied to the side wall sections in the lengthwise direction, while detachment of the abdomen side section and dorsal side section and tearing can be prevented even when large force is applied along the widthwise direction. If the lengthwise dimension is less than 50 µm, detachment (by force in the widthwise direction) will be more likely to occur when the article is worn, while if it is greater than 200 µm, it may be difficult to accomplish detachment during removal. If the widthwise dimension is less than 100 µm, detachment will be more likely to occur when the article is worn, and if it is greater than 400 µm, detachment (by force in the lengthwise direction) will be more difficult during removal.

The absorbent article of the present invention may also be (7) the absorbent article according to (6) above, wherein a pair of joint sections including the joint section are joined together respectively with both edge sections in the widthwise direction of the abdomen side section and the dorsal side section overlapping in the thickness direction along the lengthwise direction, both of the pair of joint sections respectively include the plurality of fused joint sections, and a difference between a dimension in the widthwise direction (one side) of the side wall section at one joint section of the pair of joint sections and a dimension in the widthwise direction (one side) of the side wall sections at the other joint section is within 40 µm.

In this absorbent article, the dimension of the side wall section in the widthwise direction (the thickness of the cylinder in the widthwise direction) is approximately the same dimension at one joint section (for example, the joint section on the left side) and the other joint section (for example, the joint section on the right side) in the widthwise direction. Therefore, when the wearer attempts to remove the absorbent article, the left and right joint sections can both be detached with approximately the same force. This allows the wearer to easily detach the left and right joint sections.

The absorbent article of the present invention may also be (8) the absorbent article according to (6) or (7) above, wherein a difference between an inner side dimension and an outer side dimension of the side wall section of each of the pair of joint sections in the widthwise direction is within 40 µm.

With this absorbent article, the inner side dimension (cylinder thickness) and the outer side dimension (cylinder thickness) in the widthwise direction at each of the side wall sections are approximately the same dimension. At each of the side wall sections, therefore, the force necessary to detach the inner side section and the force necessary to detach the outer side section in the widthwise direction are approximately the same. Therefore when the wearer attempts to remove the absorbent article, it is possible to prevent the direction of force detaching the abdomen side section and dorsal side section from being in a direction diagonal to the lengthwise direction or in the widthwise direction, and therefore tearing of the abdomen side section or dorsal side section in a direction diagonal to the lengthwise direction or in the widthwise direction can be minimized.

The absorbent article of the present invention may also be (9) the absorbent article according to any one of (1) to (8) above, wherein the fused section is located inside the side wall section further toward the abdomen side section than the dorsal side section in the thickness direction.

In this absorbent article, the fused section is located inside the cylindrical side wall section further toward the abdomen side section in the thickness direction. Thus, when a wearer bows down to detach the abdomen side section and dorsal side section of their own disposable pants article, the fused sections are more easily visible, and therefore the wearer can pinch the area near the fused joint sections with the fingers in a more reliable manner while detaching them. This will allow the abdomen side section and dorsal side section of the joint sections to be more easily detached.

The absorbent article of the present invention may also be (10) the absorbent article according to any one of (1) to (9) above, wherein at the joint section, an edge in the lengthwise direction on the abdomen side section and an edge in the lengthwise direction om the dorsal side section are mutually deviating in the lengthwise direction.

In this absorbent article, the edge in the lengthwise direction of the abdomen side section and the edge of the dorsal side section mutually deviate from each other in the lengthwise direction. Therefore when the wearer attempts to remove the absorbent article, the wearer can more easily pinch the edge of the abdomen side section with one hand, while pinching the edge of the dorsal side section with the other hand. This allows the abdomen side section and dorsal side section of the joint section to be more easily detached.

### [ADVANTAGEOUS EFFECTS OF INVENTION]

According to the present invention, it is possible to provide an absorbent article in which separation between the abdomen side section and dorsal side section is difficult while it is being worn, but separation in the lengthwise direction is easy when it is to be removed.

### BRIEF DESCRIPTION OF DRAWINGS

FIG. 1 is a perspective view showing an example of a construction of a disposable diaper according to an embodiment.
FIG. 2 is a plan view of the disposable diaper of FIG. 1 in an expanded state.
FIG. 3 is a plan view showing an example of a construction of a joint section of the disposable diaper of FIG. 1.
FIG. 4 is a schematic view for explaining an example of a construction of the joint section of FIG. 3.
FIG. 5 is a plan view and perspective view showing a construction of a fused joint section of the joint section of FIG. 4.
FIG. 6 is a schematic view for explaining a state during detachment of the fused joint section in FIG. 5.
FIG. 7 is a schematic view for explaining a method of forming the joint section in the disposable diaper of FIG. 1.
FIG. 8 is a schematic view for explaining another example of a construction of the joint sections of FIG. 3.
FIG. 9 is a plan view showing a construction of a fused joint section of the joint sections of FIG. 8.
FIG. 10 is a pair of SEM photographs showing a cross-section of a fused joint section according to an Example.
FIG. 11 is a graph showing a relationship between shape and strength of fused joint sections.
FIG. 12 is a graph showing a relationship between shape and strength of fused joint sections.
FIG. 13 is a graph showing a relationship between shape and strength of fused joint sections.

### DESCRIPTION OF EMBODIMENTS

An absorbent article according to an embodiment of the present invention will now be explained using a pants-type disposable diaper (hereunder also referred to simply as "disposable diaper") as an example. However, the absorbent article of the present invention is not limited to this example, and the present invention may be applied to various types of absorbent articles, without departing from the scope of the subject matter of the present invention. Examples of absorbent articles include "three-piece type" and "two-piece type" disposable diapers, and disposable diapers having a joint section formed on either the right or left side section of the waist opening, and having a joining (or engaging) means for mutual joining (or engaging) formed on the other side section, without having a joint section.

### (First embodiment)

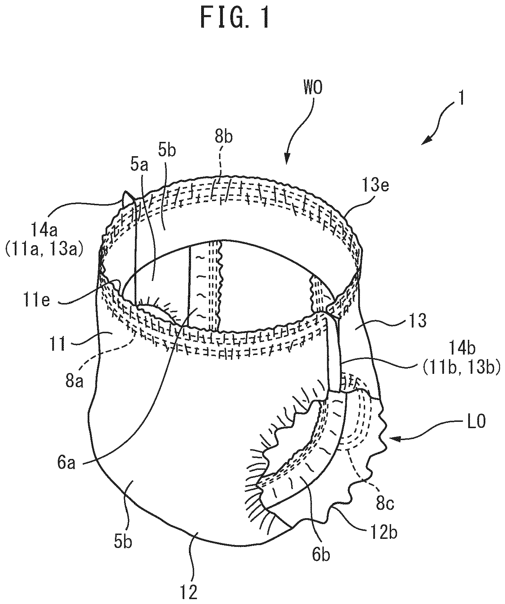

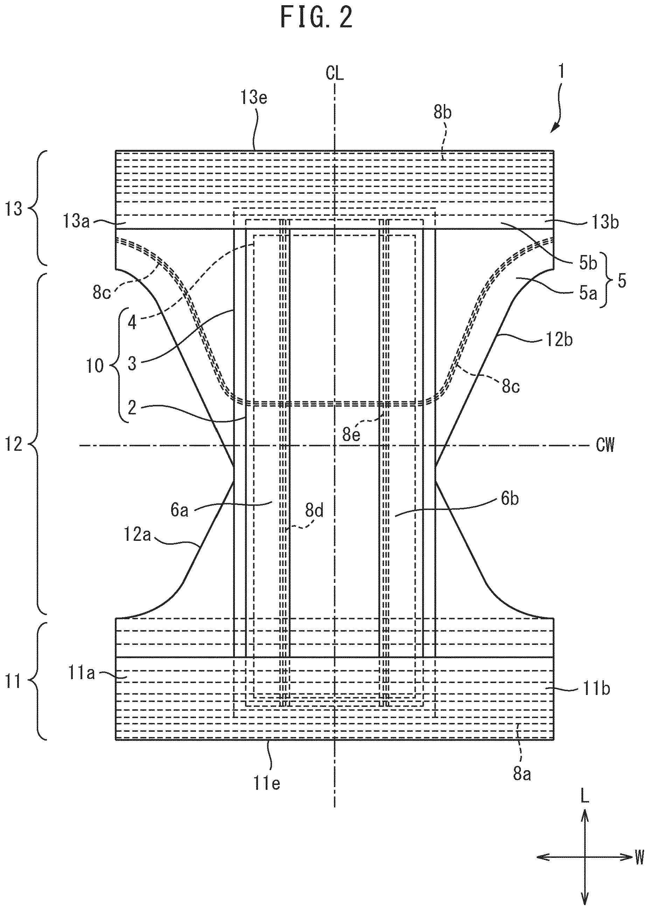

Fig. 1 and Fig. 2 are views showing a disposable diaper 1 according to this embodiment. Fig. 1 is a perspective view showing the construction of the disposable diaper 1, and Fig. 2 is a plan view showing the state of the disposable diaper 1 when it is expanded. In the state shown in Fig. 2, the disposable diaper 1 has a lengthwise direction L, a widthwise direction W perpendicular to the lengthwise direction L, and a thickness direction T perpendicular to the lengthwise direction L and the widthwise direction W, and also has a center axis line CL running through the center of the disposable diaper 1 in the widthwise direction W and extending in the lengthwise direction L, and a center axis line CW running through the center of the disposable diaper 1 in the lengthwise direction L and extending in the widthwise direction W. The direction toward and the direction away from the center axis line CL are the direction on the inner side and the direction on the outer side, respectively, in the widthwise direction W. The direction toward and the direction away from the center axis line CW are the direction on the inner side and the direction on the outer side, respectively, in the lengthwise direction L. The view in the thickness direction T of the disposable diaper 1 from above in the perpendicular direction when it is on a horizontal plane, will be referred to as the "plan view", and the shape as seen in the plan view will be referred to as the "planar shape". The terms "skin side" and "non-skin side" refer, respectively, to the side relatively near the skin and the side relatively far from the skin of the wearer in the thickness direction T of the disposable diaper 1 when the disposable diaper 1 is worn. The lengthwise direction L, widthwise direction W and thickness direction T shown in Fig. 2 will also be used for the respective directions of the disposable diaper 1 in the state shown in Fig. 1.

The disposable diaper 1 is a pants-type diaper including an abdomen side section 11, a dorsal side section 13, and a middle section 12 between the abdomen side section 11 and dorsal side section 13. The abdomen side section 11 is the portion of the disposable diaper 1 that contacts the abdominal region of the wearer. The middle section 12 is the portion of the disposable diaper 1 that contacts the crotch region of the wearer. The dorsal side section 13 is the portion of the disposable diaper 1 that contacts the gluteal region and/or dorsal region of the wearer. Both edge sections 11a, 11b in the widthwise direction W of the abdomen side section 11 and both edge sections 13a, 13b in the widthwise direction W of the dorsal side section 13 are joined together at a pair of joint sections 14a, 14b while overlapping in the thickness direction T along the lengthwise direction L. In the disposable diaper 1, a waist opening WO is formed by the edge section 11e of the abdomen side section 11 at the side opposite from the middle section 12 in the lengthwise direction L, and the edge section 13e of the dorsal side section 13 at the side opposite from the middle section 12 in the lengthwise direction L. A pair of leg openings LO, LO are also formed in the disposable diaper 1 by both side sections 12a, 12b of the middle section 12 in the widthwise direction W.

The directions of the disposable diaper 1 in the state shown in Fig. 1 are as follows. The lengthwise direction L is the direction along the outer shape of the disposable diaper 1, running from the abdomen side section 11 (or dorsal side section 13) through the middle section 12 to the dorsal side section 13 (or abdomen side section 11). The widthwise direction W is the direction along the outer shape of the disposable diaper 1, running along the surface of the joint section 14a (or joint section 14b), reaching from the inside edge of the joint section 14a (or joint section 14b) along the peripheral center section of the abdomen side section 11 or dorsal side section 13, up to the inside edge section of the joint section 14b (or joint section 14a), along the surface of the joint section 14b (or joint section 14a). The thickness direction T is the direction perpendicular to the plane in contact with the outer surface of the disposable diaper 1, i.e. to the tangent plane. For each of the pair of joint sections 14a, 14b, the lengthwise direction, widthwise direction and thickness direction in the state shown in Fig. 1 are the same as the lengthwise direction L, widthwise direction W and thickness direction T in the state shown in Fig. 2.

The disposable diaper 1 includes an absorbent body 10. The absorbent body 10 includes a liquid-permeable top sheet 2, a liquid-impermeable back sheet 3, and an absorbent member 4 situated between the top sheet 2 and back sheet 3. The top sheet 2 may be, for example, a liquid-permeable nonwoven fabric or woven fabric, a liquid-permeable pore-formed synthetic resin film, or a composite sheet thereof. The back sheet 3 may be, for example, a liquid-impermeable nonwoven fabric or synthetic resin film, a composite sheet thereof, an SMS nonwoven fabric, or the like. For this embodiment, the absorbent member 4 includes an absorbent core and a core wrap enveloping the absorbent core. The absorbent member 4 may be pulp fiber, synthetic fiber, an absorbing polymer or the like. The absorbent member 4 and the top sheet 2 and back sheet 3 are each joined with an adhesive, the top sheet 2 and back sheet 3 being joined by an adhesive at their peripheries. The adhesive used may be a publicly known material commonly used in disposable diapers, such as a thermoplastic adhesive.

The disposable diaper 1 further includes a liquid-impermeable cover sheet 5. For this embodiment, the cover sheet 5 includes a cover sheet 5a situated on the skin side and a cover sheet 5b situated on the non-skin side, which are laminated together in the thickness direction T and joined by an adhesive or the like. Also for this embodiment, both edge sections of the cover sheet 5b in the lengthwise direction L are folded over to the skin side so as to cover both edge sections of the cover sheet 5a in the lengthwise direction L. The cover sheets 5b, 5b at the folded locations on the abdomen side section 11 and dorsal side section 13 form the edge section 11e of the abdomen side section 11 and the edge section 13e of the dorsal side section 13, respectively. On the skin side surface of the cover sheet 5, the absorbent body 10 is placed with the top sheet 2 facing the skin side. In the disposable diaper 1, the non-skin side surface of the disposable diaper 1, i.e. the outer side, is formed by the cover sheet 5b, and the skin side surface of the disposable diaper 1, i.e. the inner side, is formed by the top sheet 2 and the cover sheet 5b at the edge section 11e and edge section 13e. The material of the cover sheet 5 may be any desired liquid-impermeable sheet, such as, for example, a liquid-impermeable nonwoven fabric or synthetic resin film, a composite sheet thereof, or an SB nonwoven fabric or SMS nonwoven fabric. Examples of materials for the cover sheet 5 include polyolefin-based materials such as polypropylene or polyethylene. The basis weight of the cover sheet 5 may be 5 to 100 g/m², for example, and it is preferably 10 to 50 g/m². The dimension of the cover sheet 5 in the thickness direction T (thickness) may be 0.2 to 5 mm, for example, and it is preferably 0.2 to 2 mm. The cover sheet 5 may be a single sheet, and it does not need to be folded.

The disposable diaper 1 further includes a liquid-impermeable pair of anti-leakage walls 6a, 6b and elastic members 8 (8a, 8b, 8c, 8d, 8e). The pair of anti-leakage walls 6a, 6b are arranged along the lengthwise direction L, on both sides of the top sheet 2 in the widthwise direction W. The elastic member 8a and elastic member 8b extend in the widthwise direction W between the cover sheet 5a and cover sheet 5b on the abdomen side section 11 and dorsal side section 13, respectively, and are situated and held with intervals between them in the lengthwise direction L. The elastic members 8a, 8b allow expansion and contraction of the waist opening WO. Elastic members 8c are disposed in a continuous manner mainly along the lengthwise direction L, at both edge sections in the widthwise direction W of the portion of the middle section 12 on the dorsal side section 13, and along the widthwise direction W at the center section of the middle section 12. The elastic members 8c allow expansion and contraction of the pair of leg openings LO, LO, respectively. At the inner edge sections of the pair of anti-leakage walls 6a, 6b in the widthwise direction W, there are disposed an elastic member 8d and an elastic member 8e, each extending in the lengthwise direction L. The elastic members 8d, 8e allow expansion and contraction of the anti-leakage walls 6a, 6b, respectively. Rubber thread is an example for the elastic members 8.

The construction of each of the pair of joint sections 14a, 14b will now be described. The joint section 14a and joint section 14b have the same basic construction, and therefore primarily the joint section 14a will be explained below.

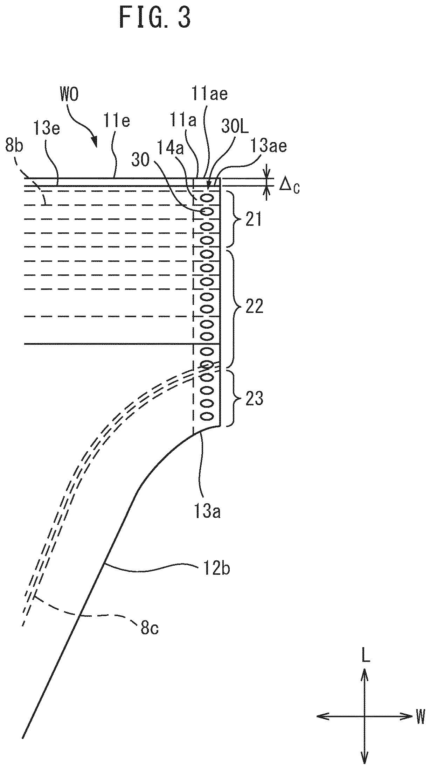

Fig. 3 is a plan view schematically showing an example of the construction of joint section 14a in a disposable diaper 1. Fig. 3 shows the joint section 14a of the disposable diaper 1 of Fig. 1 as seen from the dorsal side section 13. The joint section 14a is formed by having the edge section 11a of the abdomen side section 11 in the widthwise direction W and the edge section 13a of the dorsal side section 13 in the widthwise direction W overlapping and joined in the thickness direction T along the lengthwise direction L. There are no particular restrictions on the dimensions of the joint section 14a in the lengthwise direction L and widthwise direction W, and they may be 50 to 250 mm and 3 to 20 mm, respectively, for example. For this embodiment, at the joint section 14a, the edge section 11ae in the lengthwise direction L of the edge section 11a on the abdomen side section 11 and the edge section 13ae in the lengthwise direction L of the edge section 13a on the dorsal side section 13 are joined together with deviation of a prescribed length Δ_{C} in the lengthwise direction L. The size of Δ_{C} may be ±0.5 to 10 mm, for example. The joint section 14a includes a plurality of fused joint sections 30. Specifically, the edge section 11a and the edge section 13a are joined by at least a plurality of fused joint sections 30. For this embodiment, the fused joint sections 30 are formed by an ultrasonic seal method. All or parts of the sections of the joint section 14a other than those where the plurality of fused joint sections 30 are formed may be joined by an adhesive (such as a hot-melt adhesive), for example.

For this embodiment, the plurality of fused joint sections 30 are disposed along the lengthwise direction L, with intervals between them. That is, the joint section 14a has a pressure welded section row 30L where the plurality of fused joint sections 30 are arranged along the lengthwise direction L, with intervals between them. The pressure welded section row 30L at the joint section 14a is a single row for this embodiment. However, the pressure welded section row 30L is not limited to being a single row, and multiple rows may be disposed entirely or partially in the widthwise direction W with intervals between them. For this embodiment, the intervals between the fused joint sections 30 are constant. However, the intervals between the fused joint sections 30 may vary instead depending on the location.

The joint section 14a has, in the lengthwise direction L, a waist side edge region 21 that includes the edge section near to the waist opening WO, a leg side edge section region 23 that includes the edge section far from the waist opening WO and near to the leg opening LO, and a center region 22 between the waist side edge region 21 and the leg side edge region 23. The waist side edge region 21 and leg side edge region 23 are regions in a range of 1/8 to 1/3 of the joint section 14a from the edge sections of the waist opening WO and leg opening LO, respectively, in the lengthwise direction L, while the center region 22 is the remaining region. However, the waist side edge region 21 and leg side edge region 23 do not necessarily have to have identical lengths in the lengthwise direction L. For this embodiment, they are each regions within a range of 1/5. For this embodiment, the waist side edge region 21, center region 22 and leg side edge region 23 have one pressure welded section row 30L along the lengthwise direction L. However, each region may have multiple pressure welded section rows 30L, or they may partially have one pressure welded section row 30L and partially have multiple pressure welded section rows 30L.

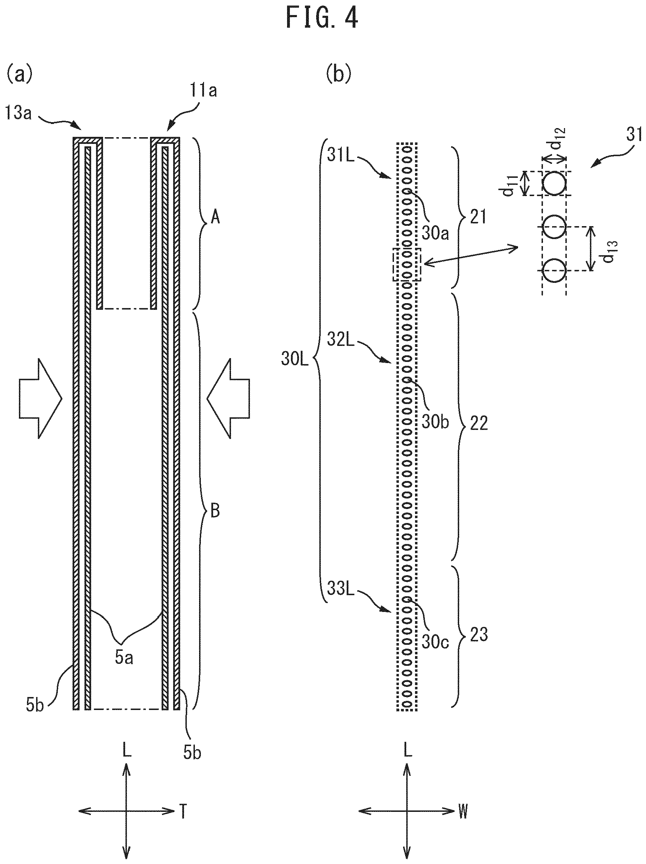

Fig. 4 is a schematic view for explaining the construction of the joint section 14a of Fig. 3. In this drawing, however, the elastic members 8a, 8b, 8c are omitted. Fig. 4(a) is a schematic view of the joint section 14a as seen from the edge section in the widthwise direction W. This diagram shows an example of the layered state of the cover sheet 5 at the joint section 14a. For this embodiment, at the edge section 11a of the abdomen side section 11, the edge section on the waist opening WO side in the lengthwise direction L of the cover sheet 5b is folded over to the skin side so as to cover the edge section in the lengthwise direction L of the cover sheet 5a. Thus, the edge section 11a has region A where a three-layer cover sheet is layered, including the cover sheet 5b, the cover sheet 5a and the folded cover sheet 5b, and a region B where a two-layer cover sheet is layered, including the cover sheet 5a and the cover sheet 5b. Similarly, at the edge section 13a of the dorsal side section 13, the edge section on the waist opening WO side in the lengthwise direction L of the cover sheet 5b is folded over to the skin side so as to cover the edge section in the lengthwise direction L of the cover sheet 5a. The edge section 13a thus has a region A where a three-layer cover sheet is layered and a region B where a two-layer cover sheet is layered. Also, as indicated by the outline arrows in the drawing, the edge section 11a and edge section 13a are layered so as to overlap in the thickness direction T and are joined, thus constructing the joint section 14a. For this embodiment, the edge section 11a and edge section 13a joint together the respective regions A and respective regions B. The size of region A is not particularly restricted and may be 10 mm to 100 mm, for example. There are no particular restrictions on the number of layers of the cover sheet 5 on the abdomen side section 11 and dorsal side section 13, and it may be a single layer or multiple layers, such as two or more layers. The cover sheet 5 also does not need to be folded.

The edge section 11a and the edge section 13a at the joint section 14a are joined by at least a plurality of fused joint sections 30. Fig. 4(b) is a schematic view of the joint section 14a as seen from the dorsal side section 13 in the thickness direction T. This diagram shows an example of an arrangement of the fused joint sections 30. For this embodiment, one pressure welded section row 31L, pressure welded section row 32L and pressure welded section row 33L are disposed in the waist side edge region 21, the center region 22 and the leg side edge region 23, respectively, as pressure welded section rows 30L. In the pressure welded section row 31L, pressure welded section row 32L and pressure welded section row 33L, a plurality of fused joint sections 30a, a plurality of fused joint sections 30b and a plurality of fused joint sections 30c, respectively, are arranged along the lengthwise direction L. For this embodiment, the fused joint sections 30a, 30b and 30c of the respective pressure welded section rows 31L, 32L and 33L have the same planar shapes. In other words, when the fused joint sections 30a, 30b and 30c are formed by an ultrasonic seal method, the same planar shape 31 is used for the tip section of the ultrasonic horn for the fused joint sections 30a, 30b and 30c. For this embodiment, the planar shape 31 of the tip section of the ultrasonic horn is circular as shown in the diagram, and the dimension d₁₁ in the lengthwise direction L is equal to the dimension d₁₂ in the widthwise direction W (circular diameter). The size of d₁₁ = d₁₂ is not particularly restricted, and it may be 0.5 mm to 5 mm, for example. The distance d₁₃ between adjacent fused joint sections 30a in the lengthwise direction L (the distance between their circle centers) is not particularly restricted, and it may be 1 mm to 30 mm. The reason for this is described below.

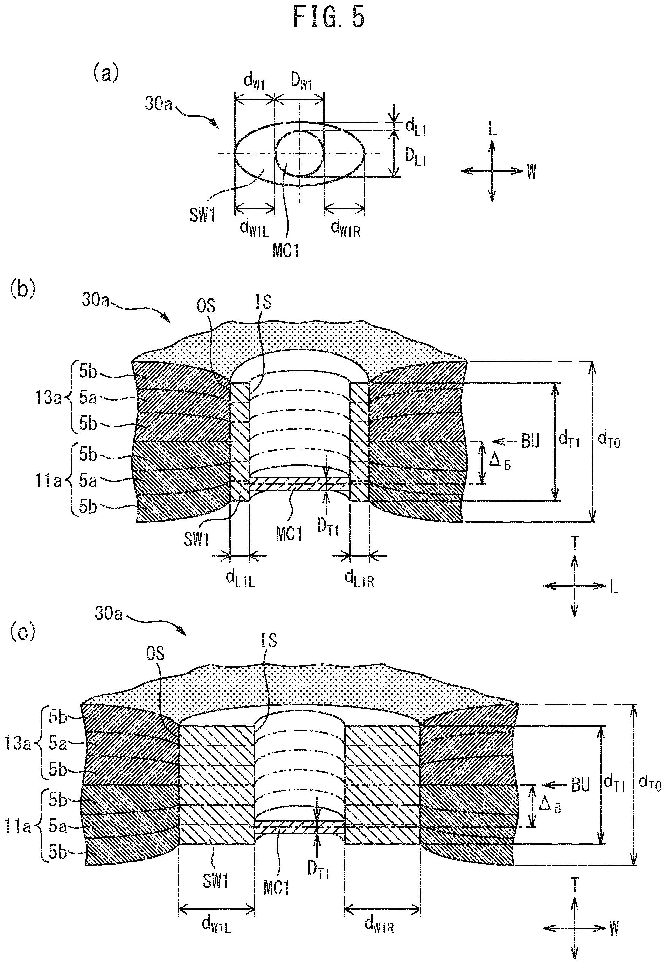

The details regarding the fused joint section 30 will now be explained. Fig. 5 is a plan view and a perspective view showing an example of the construction of a fused joint section 30a of the joint section 14a. Fig. 5(a) is a plan view, Fig. 5(b) is a perspective view including a cross-section as seen from the widthwise direction W, and Fig. 5(c) is a perspective view including a cross-section as seen from the lengthwise direction L. The construction for the fused joint section 30a will be described here, but the other fused joint sections 30b and 30c of this embodiment have the same construction.

The fused joint section 30a includes a fused section MC1 and a side wall section SW1. The fused section MC1 has the plurality of cover sheets 5a, 5b on the abdomen side section 11 and the plurality of cover sheets 5a, 5b on the dorsal side section 13 fused in the thickness direction T. For this embodiment, the fused section MC1 is formed by fusion of the plurality of cover sheets 5a, 5b by energy supplied from the tip section of the ultrasonic horn, and compression in the thickness direction T by the tip section. The fused section MC1 is therefore very firmly joined in the thickness direction T. In addition, the dimension (thickness) D_{T1} of the fused section MC1 in the thickness direction T is significantly smaller than the dimension d_{TO} in the thickness direction T of the plurality of cover sheets 5a, 5b that are merely layered on the abdomen side section 11 and dorsal side section 13. The size of d_{TO} may be 200 to 5000 µm, for example, and the size of D_{T1} may be 15 to 100 µm, for example. If the size of D_{T1} is less than 15 µm, fusion between the cover sheets will be unstable and perforations will tend to be generated during fusion. If the size of D_{T1} is greater than 100 µm, there will be locations where fusion is incomplete, and such locations will require extra tearing. The planar shape of the fused section MC1 is approximately the same as the planar shape 31 of the tip section of the ultrasonic horn. That is, the dimension D_{L1} in the lengthwise direction L and the dimension D_{W1} in the widthwise direction W are approximately the same as the dimension d₁₁ in the lengthwise direction L and the dimension d₁₂ in the widthwise direction W, of the planar shape 31 of the tip section. For this embodiment, the planar shape of the fused section MC1 is circular as shown in the diagram, but the sizes of D_{L1} and D_{W1} are not particularly restricted and may be 0.5 mm to 5 mm, for example. If D_{L1} and D_{W1} (i.e. d₁₁ and d₁₂) are less than 0.5 mm, then the absolute amount of the side wall section will be reduced, and therefore during tearing, force will be applied over the entirety instead of gradually from the pattern of the fused sections, thus making it more likely for them to be detached (by force along the widthwise direction W) while being worn. If D_{L1} and D_{W1} are greater than 5 mm, the absolute amount of side wall section will be increased, potentially making it difficult to detach them during removal. Moreover, the distance between adjacent tip sections of the ultrasonic horns (that is, d₁₃) is 1 mm to 30 mm, and correspondingly the distance between adjacent fused sections MC1 is also 1 mm to 30 mm. If the distance is less than 1 mm, there will be more fused joint sections on the tearing surface, potentially making it difficult to detach them (with force along the lengthwise direction) during removal. If it exceeds 30 mm, conversely, fewer fused joint sections will be present, potentially making it more likely for them to be detached when worn.

The side wall section SW1, on the other hand, has the plurality of cover sheets 5a, 5b on the abdomen side section 11 and the plurality of cover sheets 5a, 5b on the dorsal side section 13 fused together in the thickness direction T, from the perimeter edge of the fused section MC1 along the cylindrical shape in the thickness direction T. For this embodiment, the side wall section SW1 is formed by fusion of the plurality of cover sheets 5a, 5b by energy supplied from the tip section of the ultrasonic horn or by heat of the fused section MC1, while it is not compressed by the tip section, buy it is dragged and slightly compressed in the thickness direction T due to compression of the fused section MC1. Therefore, the side wall section SW1 is more weakly joined in the thickness direction T than the fused section MC1. The dimension (thickness) d_{T1} of the side wall section SW1 in the thickness direction T is smaller than the dimension d_{TO} in the thickness direction T of the plurality of cover sheets 5a, 5b that are merely layered on the abdomen side section 11 and dorsal side section 13, but it is larger than the dimension D_{T1} of the fused section MC1 in the thickness direction T. At the fused joint sections 30a, therefore, d_{TO} > d_{T1} > D_{T1}. The size of d_{T1} may be 100 to 1000 µm, for example. If the size of d_{T1} is less than 100 µm, fusion will proceed too far, making it difficult to accomplish tearing. If the size of d_{T1} exceeds 1000 µm, non-fused spots will be created uniformly in the fused sections and the tearing starting points will be unstable. The cylindrical side wall section SW1 has an outer surface OS forming the outer side surface of the cylinder, and an inner surface IS forming the inner side surface of the cylinder. The outer surface OS and inner surface IS are the surfaces on the outer side and inner side of the fused site established from the perimeter edge of the fused section MC1 in the thickness direction T, as observed with a scanning electron microscope, for example. However, the outer surface OS and inner surface IS do not need to be mutually parallel or corresponding shapes, they may even be irregular surfaces instead of smooth surfaces, they may be surfaces with fibers jutting out from the surface, and they also do not need to be crossing perpendicularly with respect to the surface of the fused section MC1. A cylindrical shape is one that is narrow, long and hollow, also referred to as tubular. The cylindrical shape may be bent or meandering so long as it extends along the thickness direction T, and the cross-sectional shape perpendicular to the thickness direction T may be circular, elliptical, polygonal or a combination of such shapes, while the shape and area of the cross-section perpendicular to the thickness direction T also do not need to be constant in the thickness direction T. With such shapes, the cylindrical side wall section SW1 of the fused joint section 30a becomes torn when the joint section 14a is removed.

With such a fused joint section 30a, the location of the fused section MC1 in the thickness direction T deviates from the border BU between the abdomen side section 11 and dorsal side section 13, toward the abdomen side section 11 or dorsal side section 13, deviating by at least Δ_{B}. However, the location of the fused section MC1 in the thickness direction T is the location of the center of the fused section MC1 in the thickness direction T, and when the fused section MC1 is not flat, it is the center of the fused section MC1 in the thickness direction T at the bonded section between the fused section MC1 and the side wall section SW1 in the lengthwise direction L. In the example shown in Fig. 5, for example, the location of the fused section MC1 in the thickness direction T deviates from the border BU between the plurality of cover sheets 5a, 5b on the abdomen side section 11 and the plurality of cover sheets 5a, 5b on the dorsal side section 13, by Δ_{B} on (the edge section 11a of) the abdomen side section 11. Here, Δ_{B} is a value at least exceeding 1/2 of the dimension D_{T1} of the fused section MC1 in the thickness direction T, and it is a value of no greater than then dimension of the laminate of the cover sheet 5b and cover sheet 5a in the thickness direction T. The size of Δ_{B} may be 10 to 500 µm, for example. The plane including the border BU does not cross with the fused section MC1.

Also, in the fused joint section 30a, the dimension d_{L1} in the lengthwise direction L of the side wall section SW1 (the thicknesses of the cylinder in the lengthwise direction, D_{L1L} and D_{L1R}) is smaller than the dimension d_{W1} in the widthwise direction W (the thicknesses of the cylinder in the widthwise direction, d_{W1L} and d_{W1R}) (d_{L1} < d_{W1}). However, the dimension d_{L1} in the lengthwise direction L of the side wall section SW1 (the thickness of the cylinder in the lengthwise direction) is the width in the lengthwise direction L of the cross-section, when the side wall section SW1 has an imaginary cut at a cross-section running through the center of gravity of the planar shape of the fused section MC1 and perpendicular to the widthwise direction W (i.e. it is the width of the cross-section of the fused section MC1 on one side, among the cross-sections on both sides in the lengthwise direction L). When the width is not constant in the thickness direction T, it is the width at the border BU. The size of d_{L1} may be 50 to 200 µm, for example. For this embodiment, the widths of the cross-section of the fused section MC1 on both sides in the lengthwise direction L are approximately equal. The dimension d_{W1} in the widthwise direction W of the side wall section SW1 (the thickness of the cylinder in the widthwise direction) is the width in the widthwise direction W of the cross-section, when the side wall section SW1 has an imaginary cut at a cross-section running through the center of gravity of the planar shape of the fused section MC1 and perpendicular to the lengthwise direction L (i.e. it is the width of the cross-section of the fused section MC1 on one side, among the cross-sections on both sides in the widthwise direction W). When the width is not constant in the thickness direction T, it is the width at the border BU. The size of d_{W1} may be 100 to 400 µm, for example. For this embodiment, the widths of the cross-section of the fused section MC1 on both sides in the widthwise direction W are approximately equal. Therefore, the cylindrical side wall section SW1 of the fused joint section 30a has small widths in the lengthwise direction L, i.e. they are thin and relatively easy to tear, against force from the lengthwise direction L, while having large widths in the widthwise direction W, i.e. being thick and relatively resistant to tearing, against force from the widthwise direction W.

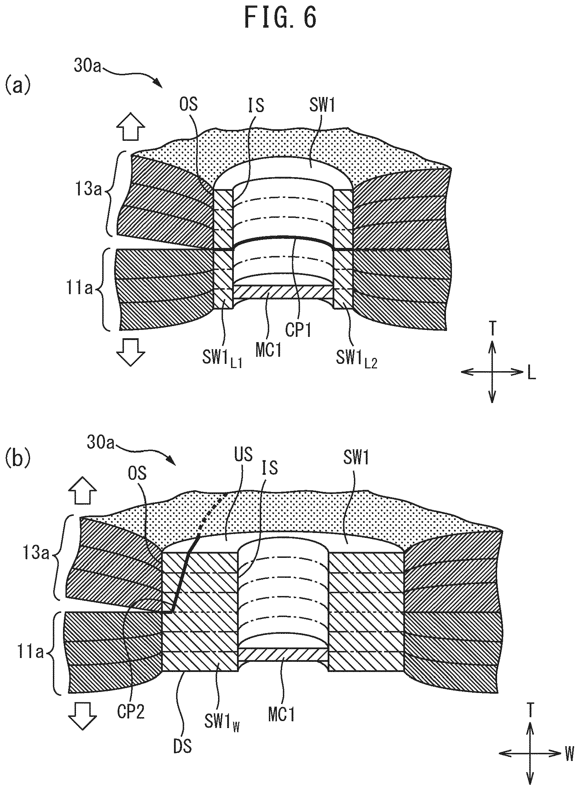

The function and effect of joint section 14a having such a construction will now be described. Fig. 6 is a schematic view for explaining the state during detachment of the fused joint section 30 in Fig. 5. As mentioned above, each fused joint section 30a of the joint section 14a includes a fused section MC1 and a side wall section SW1 extending from the perimeter edge of the fused section MC1 along the cylindrical shape in the thickness direction T, and the dimension in the lengthwise direction L of the side wall section SW1 (the thickness of the cylinder in the lengthwise direction) d_{L1} is smaller than the dimension in the widthwise direction W (the thickness of the cylinder in the widthwise direction) d_{W1}. The section of the cylinder of the side wall section SW1 in the lengthwise direction L is therefore easy to tear, while the section in the widthwise direction W is difficult to tear. In addition, the dimension d_{T1} (thickness) of the side wall section SW1 in the thickness direction T is larger than the dimension D_{T1} (thickness) of the fused section MC1 in the thickness direction T. At the fused section MC1, therefore, the plurality of sheets (cover sheets 5a, 5b) are joined together relatively firmly, while at the side wall section SW1, the plurality of sheets (cover sheets 5a, 5b) are joined relatively weakly. In addition, since the location of the fused section MC1 in the thickness direction T deviates from the border BU between the abdomen side section 11 and dorsal side section 13, the fused section MC1 is not included at the location of separation when the abdomen side section 11 and dorsal side section 13 are separated.

Therefore, when the (edge section 11a of the) abdomen side section 11 of the joint section 14a and the (edge section 13a of the) dorsal side section 13 are detached along the lengthwise direction 1, as shown in Fig. 6(a), tearing can be initiated at the section SW1_{L} in the lengthwise direction L, which is the side wall section SW1 that is relatively weakly joined and is the portion of the side wall section SW1 that is easily torn. This will allow the abdomen side section 11 and dorsal side section 13 to be easily detached along the interface CP1 between them. In other words, the abdomen side section 11 and the dorsal side section 13 are not detached at the strongly joined fused sections MC1 and their surrounding regions. In this disposable diaper 1, therefore, it is possible to detach the abdomen side section 11 and dorsal side section 13 of the joint section 14a with relatively small force.

Specifically, it is possible to easily detach (tearing can be initiated between) the abdomen side section 11 and dorsal side section 13 along their interface CP1 with a relatively small force, at the sections SW1_{L1} on one side of the side wall section SW1 in the lengthwise direction L, from the outer side surface OS of the cylinder up to the inner side surface IS of the cylinder of the side wall section SW1. In addition, if the abdomen side section 11 and dorsal side section 13 can be detached once at the sections SW1_{L1} of the side wall section SW1, from the outer side surface OS of the cylinder to the inner side surface IS of the cylinder, then it is possible to easily detach the abdomen side section 11 and dorsal side section 13 along their interface CP1 at the section SW1_{L2} on the other side in the lengthwise direction L, from the inner side surface IS of the cylinder to the outer side surface OS of the cylinder of the side wall section SW1. This will allow the abdomen side section 11 and dorsal side section 13 of the joint section 14a to be easily detached (to be torn).

When the wearer is wearing the disposable diaper 1, on the other hand, force applied to the joint section 14a in response to movement of the wearer mainly moves in the widthwise direction W. Moreover, in the disposable diaper 1, the dimension d_{W1} of the side wall section SW1 in the widthwise direction W of the cylinder (the thickness of each cylinder in the widthwise direction) is larger than the dimension d_{L1} in the lengthwise direction L (the thickness of each cylinder in the lengthwise direction). When large force has been applied to the side wall section SW1 in the widthwise direction W, therefore, the section SW1_{W} with the large dimension d_{W1} of the side wall section SW1 in the widthwise direction W absorb the force. Therefore, even if cracks CP2 have formed from the outer side surface OS of the cylinder to the inner side surface IS of the cylinder of the side wall section SW1, the cracks CP2 either go through the upper surface US (Fig. 6(b)) or lower surface DS of the side wall section SW1, or stop upon reaching the fused sections MC1 that have high joining strength, before reaching the inner side surface IS of the cylinder of the side wall sections SW1. When it is worn, therefore, detachment of the abdomen side section 11 and dorsal side section 13 of the joint section 14a, and therefore tearing of the disposable diaper 1, can be minimized.

In this disposable diaper 1, therefore, the side wall section SW1 is formed surrounding the fused section MC1 in such a manner that the dimension in the lengthwise direction L is short (d_{L1}) at the fused joint section 30a, i.e. the section in the lengthwise direction L is thin, while the dimension in the widthwise direction W is long (d_{W1}), i.e. the section in the widthwise direction W is thick. This allows the ease of detachment, i.e. the ease of separation, to be different in the lengthwise direction L and the widthwise direction W, or in other words, it can provide anisotropy for the ease of detachment (ease of separation). As a result, the disposable diaper 1 is further improved such that it is easy to separate at the time of removal and has the abdomen side section 11 and dorsal side section 13 difficult to separate when it is worn. For this embodiment, both of the pair of joint sections 14a, 14b in the disposable diaper 1 have the construction described above (for example, a plurality of fused joint sections 30a). However, the present invention is not limited to this example, and it is sufficient if at least one of the pair of joint sections 14a, 14b has the construction described above. Thus, the disposable diaper 1 as a whole is further improved such that it is easy to separate at the time of removal and has the abdomen side section 11 and dorsal side section 13 difficult to separate when it is worn, compared to when it does not include the construction described above.

For this embodiment, both of the pair of joint sections 14a, 14b in the disposable diaper 1 have the construction described above (for example, a plurality of fused joint sections 30a). However, the present invention is not limited to this example, and it is sufficient if at least one of the pair of joint sections 14a, 14b has the construction described above.

For example, the absorbent article may be one in which one of the pair of joint sections (for example, the joint section 14a) has the construction, while the other has a construction similar to that of the prior art. Thus, the disposable diaper 1 as a whole is further improved such that it is easy to separate at the time of removal and has the abdomen side section 11 and dorsal side section 13 difficult to separate when it is worn, compared to when it does not include the construction described above.

The absorbent article may also be one wherein a joint section is formed which has one of the two edge sections of the abdomen side section and dorsal side section in the widthwise direction joined together and which has the construction described above (for example, the joint section 14a), and the other is un-joined and has a joining (or engaging) means allowing the abdomen side section and dorsal side section to be joined (or engaged) together. The joining (engaging) means may be a hook-and-loop fastener, for example. In this case as well, the disposable diaper 1 as a whole is further improved such that it is easy to separate at the time of removal and having the abdomen side section 11 and dorsal side section 13 difficult to separate when it is worn, compared to when it does not include the construction described above.

An example of a method of producing the disposable diaper 1 will now be explained. In this example of a method of producing the disposable diaper 1, the method of forming the pair of joint sections 14a, 14b differs from a conventional production method. Specifically, it is as follows.

First, a conventional production method is used to form a continuous disposable diaper (not shown) wherein disposable diapers, each in the state shown in Fig. 2, are continuously connected in the widthwise direction W. However, the continuous disposable diaper has a structure in which at adjacent disposable diapers, the edge section 11b of the abdomen side section 11 and the edge section 13b of the dorsal side section 13 of one of the adjacent disposable diapers are connected to the edge section 11a of the abdomen side section 11 and the edge section 13a of the dorsal side section 13 of the other of the adjacent disposable diapers. The continuous disposable diaper is disposed and transported with the widthwise direction W of the disposable diaper parallel to the machine direction MD. In this case, the lengthwise direction L of the disposable diaper is parallel to the cross-machine direction CD. Next, while being transported in the machine direction MD, the continuous disposable diaper is folded with a folding apparatus in the lengthwise direction L along the center axis line CW of each disposable diaper. In each disposable diaper of the continuous disposable diaper, therefore, the edge section 11a of the abdomen side section 11 and the edge section 13a of the dorsal side section 13 are layered, and the edge section 11b of the abdomen side section 11 and the edge section 13b of the dorsal side section 13 are layered. The continuous disposable diaper in this state is supplied to a joint section-forming apparatus.

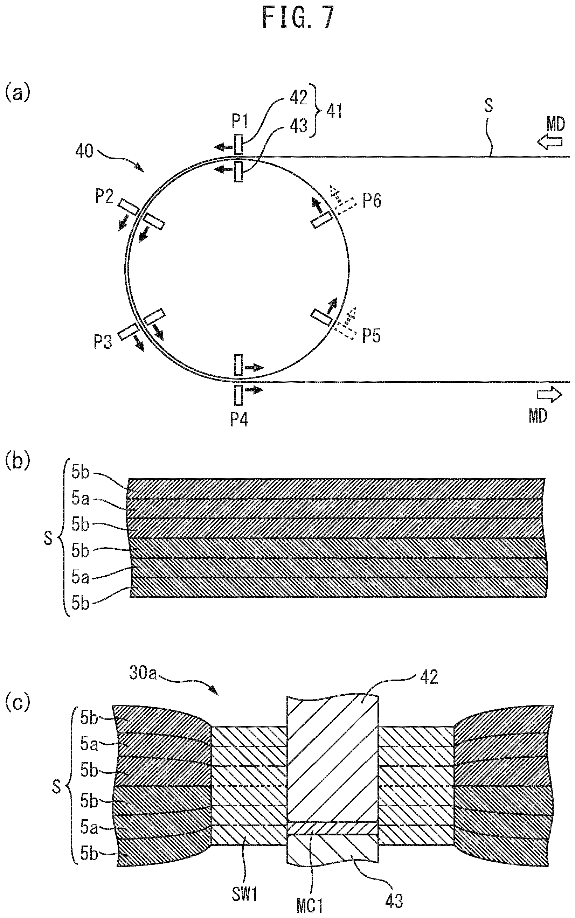

Fig. 7 is a schematic view for explaining the method of forming a joint section in the disposable diaper 1. The joint section-forming apparatus 40 is an apparatus that carries out ultrasonic sealing, and it joins together the edge section 11a of the abdomen side section 11 and the edge section 13a of the dorsal side section 13 by ultrasonic sealing, while also joining together the edge section 11b of the abdomen side section 11 and the edge section 13b of the dorsal side section 13 by ultrasonic sealing. A specific example of such an apparatus is the sealing apparatus described in Japanese Unexamined Patent Publication No. 2006-192902 (Patent Literature 3). In the joint section-forming apparatus 40, as shown in Fig. 7(a), horns 42 and anvils 43 which are the ultrasonic wave-generating means 41 and are situated on a rotating drum clamp the plurality of sheets at a position corresponding to the pair of joint sections 14a, 14b and apply ultrasonic energy for a prescribed period of time to fuse together the plurality of sheets. Each horn 42 has a tip section with a planar shape matching the planar shape of the fused section MC1.

For this embodiment, the continuous disposable diaper S is transported in the machine direction MD, while at location P1, the plurality of cover sheets 5a, 5b are clamped at a location corresponding to the pair of joint sections 14a, 14b, by the horn 42 and anvil 43. In other words, the plurality of cover sheets 5a, 5b at the edge section 11a on the abdomen side section 11 and the plurality of cover sheets 5a, 5b at the edge section 13a on the dorsal side section 13 are clamped, and the plurality of cover sheets 5a, 5b at the edge section 11b on the abdomen side section 11 and the plurality of cover sheets 5a, 5b at the edge section 13b on the dorsal side section 13 are clamped. Next, the continuous disposable diaper S is transported in the machine direction MD while ultrasonic energy is applied to the clamped plurality of cover sheets 5a, 5b. The continuous disposable diaper S is then transported in the machine direction MD while ultrasonic energy continues to be applied as it passes through location P2 and location P3. At location P4, the plurality of cover sheets 5a, 5b are released from the horn 42 and anvil 43. That is, the horn 42 and anvil 43 separate from the plurality of cover sheets 5a, 5b, and return to location P1 via location P5 and location P6. With this operation, the plurality of cover sheets 5a, 5b at locations corresponding to the pair of joint sections 14a, 14b are fused together by ultrasonic energy, and fused joint sections 30a of the pair of joint sections 14a, 14b are formed in each disposable diaper of the continuous disposable diaper S. The joint section 14a and joint section 14b of adjacent disposable diapers of the continuous disposable diaper S are then cut in the cross-machine direction CD, producing a disposable diaper 1. Fig. 7(b) shows a plurality of cover sheets 5a, 5b at a location corresponding to the pair of joint sections 14a, 14b of the continuous disposable diaper S before reaching location P1, at which point the fused joint sections 30a have not been formed. Fig. 7(c) shows a plurality of cover sheets 5a, 5b at a location corresponding to the pair of joint sections 14a, 14b of the continuous disposable diaper S when they have reached location P4, where a fused joint section 30a including a fused section MC1 and a side wall section SW1 is formed.

In this production method, the (horn 42 and anvil 43 of the) ultrasonic wave-generating means 41 clamps a plurality of cover sheets 5 and apply ultrasonic energy to the same location of each of the plurality of cover sheets 5, while moving continuously with the plurality of cover sheets 5 for a prescribed period of time. In other words, the ultrasonic wave-generating means 41 applies ultrasonic energy to the plurality of cover sheets 5 while pressing them continuously at the same location for a prescribed period of time, to fuse them together. Therefore, not only are fused sections MC1 formed at the sections to which ultrasonic energy is applied while pressing the horn 42 and anvil 43, but energy can also be adequately transmitted to the sections surrounding the fused sections MC1, fusing them and forming side wall sections SW1. The side wall sections SW1 surrounding the fused sections MC1 in this manner may be formed while controlling the size of the ultrasonic energy, the length of the prescribed time period, the pressing pressure, the shapes of the tip sections of the horns 42 and the properties of the plurality of cover sheets 5, to within suitable ranges. Depending on the production yield, some of the plurality of fused joint sections 30a may lack the construction described above.

As a preferred mode of this embodiment, each of the pair of joint sections 14a, 14b has a pressure welded section row 30L where the plurality of fused joint sections 30 are arranged along the lengthwise direction L, with intervals between them. In this case, when the disposable diaper 1 is to be removed, the abdomen side section 11 and dorsal side section 13 can be detached with relatively small force at the continuously aligned fused joint sections 30, for a relatively long distance along the lengthwise direction L. This will allow the abdomen side section 11 and dorsal side section 13 of each of the pair of joint sections 14a, 14b to be more easily detached. As a more preferred mode of this embodiment, each of the pair of joint sections 14a, 14b has only one pressure welded section row 30L as the plurality of fused joint sections 30. When it has a plurality of pressure welded section rows in the widthwise direction W, the pulling force between the abdomen side section 11 and dorsal side section 13 along the lengthwise direction L when the disposable diaper is removed is diffused at each row, and cracks produced by detachment run along the diagonal direction with respect to the lengthwise direction L or along the widthwise direction W, potentially increasing the force required for detachment. However, this disposable diaper 1 has only one pressure welded section row 30L. When the disposable diaper 1 is removed, therefore, pulling force between the abdomen side section 11 and dorsal side section 13 along the lengthwise direction L can be concentrated to the single pressure welded section row 30L. This allows the abdomen side section 11 and dorsal side section 13 of each of the pair of joint sections 14a, 14b to be more easily detached with a smaller degree of force.

As another preferred mode of this embodiment, each of the pair of joint sections 14a, 14b has a waist side edge section region 21, a leg side edge section region 23 and a center region 22, and at least the waist side edge section region 21 and the center region 22 have only one pressure welded section row 31L as the plurality of fused joint sections 30. That is, the disposable diaper 1 not only has a plurality of fused joint sections 30a arranged in a row (pressure welded section row 31L) in each waist side edge section region 21 of the pair of joint sections 14a, 14b, but also has a plurality of fused joint sections 30b arranged in a row (pressure welded section row 32L) in the adjacent center region 22. When the disposable diaper 1 is removed, therefore, pulling force between the abdomen side section 11 and dorsal side section 13 along the lengthwise direction L can be concentrated at the single row of the plurality of fused joint sections 30a, 30b, so that it can reach at least up to the center region 22 in the lengthwise direction L of the pair of joint sections 14a, 14b. This allows the abdomen side section 11 and dorsal side section 13 of the respective pair of joint sections 14a, 14b to be easily detached by a smaller degree of force, and can minimize tearing of each of the pair of joint sections 14a, 14b in the widthwise direction W or tearing of the abdomen side section 11 or dorsal side section 13 in the widthwise direction W, when the article is worn.

As another preferred mode of this embodiment, the planar shape of the fused section MC1, i.e. the planar shape 31 of the tip section of the horn 42 of the ultrasonic wave-generating means 41, is not particularly restricted so long as the shape of the side wall section SW1 is such that: dimension d_{L1} in the lengthwise direction L < dimension d_{W1} in the widthwise direction W. The shapes may be, for example, circular, semi-circular, elliptical, semi-elliptical, polygonal, or a combination of such shapes. As another preferred mode of this embodiment, the planar shape of the fused section MC1 is such that the maximum dimension D_{L1} of the fused section MC1 in the lengthwise direction L is greater than or equal to the maximum dimension D_{W1} in the widthwise direction W. In other words, the planar shape 31 of the tip section of the horn 42 is such that the maximum dimension (d₁₁) of the tip section in the cross-machine direction CD is greater than or equal to the maximum dimension (d₁₂) in the machine direction MD. Examples of such shapes include a circle, its semicircle and a square in each of which D_{L1} = D_{W1}, and an elliptical shape, its semi-elliptical shape and a rectangular shape each of which is longer in the lengthwise direction L such that D_{L1} > D_{W1}, and combinations of the foregoing. The maximum dimension D_{L1} in the lengthwise direction L is the maximum distance between two straight lines sandwiching the fused section MC1 in the lengthwise direction L, the straight lines being parallel to the widthwise direction W and in contact with the outer edges of the fused section MC1. The maximum dimension D_{W1} in the widthwise direction W, on the other hand, is the maximum distance between two straight lines sandwiching the fused section MC1 in the widthwise direction W, the straight lines being parallel to the lengthwise direction L and in contact with the outer edges of the fused section MC1. In such a disposable diaper 1, the thickness of the cylinder formed by the side wall section SW1 can thus be thinner in the lengthwise direction L and thicker in the widthwise direction W. When the disposable diaper 1 is removed, therefore, the abdomen side section 11 and dorsal side section 13 can be more easily separated at the thin sections on the lengthwise direction L side. Moreover, even when large force is applied in the widthwise direction W while the article is being worn, the force can be absorbed at the thick sections on the widthwise direction W side, thus helping to minimize detachment of the abdomen side section 11 and dorsal side section 13 of the joint sections 14a and thus tearing of the disposable diaper 1. A circular shape is preferred as the planar shape of the fused section MC1, i.e. the planar shape 31 of the tip section of the horn 42 of the ultrasonic wave-generating means 41. The thicknesses of the cylinder formed by the side wall section SW1 can thus be suitably smaller in the lengthwise direction L and suitably larger in the widthwise direction W.

For this embodiment, the sizes D_{L1}, D_{W1} of the fused section MC1, i.e. the sizes d₁₁, d₁₂ of the tip section (the diameters, in the case of a circle, for example) are not particularly restricted but are preferably 0.5 mm to 5 mm. If they are less than 0.5 mm, the joining strength at the fused joint sections 30 will be too low, potentially resulting in detachment of the joint section 14a when the article is worn. If they are greater than 5 mm, the joining strength of the fused joint sections 30 will be too great, potentially making it difficult for the joint section 14a to separate during removal. The distance between adjacent fused sections MC1 in the lengthwise direction L, i.e. the size d₁₃ of the tip section (the center distance, in the case of a circle, for example) is not particularly restricted but is preferably 1 mm to 10 mm. If it is less than 1 mm, the number of fused joint sections 30 will be too great, potentially making it difficult for the joint section 14a to separate during removal. If it is greater than 30 mm, the number of fused joint sections 30 will be too few, potentially resulting in detachment of the joint section 14a when the article is worn.

The size of the dimension d_{L1} in the lengthwise direction L of the side wall section SW1 is not particularly restricted but is preferably 50 µm to 200 µm. If it is less than 50 µm, the joint sections 14a, 14b can potentially tear in the lengthwise direction L when the article is worn (by force along the widthwise direction). If it exceeds 200 µm, the joint sections 14a, 14b may be difficult to separate during removal. The value of d_{L1} is more preferably 70 to 160 µm and even more preferably 80 to 130 µm.The size of the dimension d_{W1} in the widthwise direction W of the side wall section SW1, on the other hand, is not particularly restricted but is preferably 100 µm to 400 µm.If it is less than 100 µm, the joint sections 14a, 14b can potentially tear in the widthwise direction W when the article is worn. If it exceeds 400 µm, the joint sections 14a, 14b may be difficult to separate during removal (by force along the lengthwise direction). The value of d_{L1} is more preferably 110 to 200 µm and even more preferably 120 to 180 µm. Depending on the dimension d_{L1} in the lengthwise direction L and the dimension d_{W1} in the widthwise direction W of the side wall section SW1, it is possible to allow detachment by application of force in the lengthwise direction L while minimizing detachment or tearing even when large force is applied in the widthwise direction W when the article is worn.

According to another preferred mode of this embodiment, the ratio (d_{L1}/d_{W1}) between the dimension d_{L1} of the side wall section SW1 in the lengthwise direction L and the dimension d_{W1} of the side wall section SW1 in the widthwise direction W is preferably 0.2 to 0.8. If the dimension d_{L1} in the lengthwise direction L and the dimension d_{W1} in the widthwise direction W of the side wall section SW1 satisfy this ratio, it will be possible to carry out detachment by application of force in the lengthwise direction L while minimizing detachment or tearing in the widthwise direction W, even when large force is applied. If it is less than 0.2 or greater than 0.8, the joint sections 14a, 14b may tear in the lengthwise direction L when the article is worn, or it may not be possible to detach them in the widthwise direction W during removal. The ratio d_{L1}/d_{W1} is more preferably 0.5 to 0.8. If the planar shape of the fused section MC1, i.e. the planar shape 31 of the tip section of the horn 42 of the ultrasonic wave-generating means 41, is a circle, then it will be preferable as d_{L1}/d_{W1} can more easily be within the preferred range.

For this embodiment, the joining strength when one fused joint section 30a is detached in the lengthwise direction L is preferably 0.5 N to 5 N. If it is less than 0.5 N, detachment may take place by force in the widthwise direction W when the article is worn, and if it is greater than 5 N, it may not be possible to detach it by force in the lengthwise direction L during removal. The joining strength when detaching one fused joint section 30a in the widthwise direction W, on the other hand, is preferably 1.0 N to 10 N. If it is less than 1.0 N, detachment may take place by force in the widthwise direction W when the article is worn, and if it is greater than 10 N, it may not be possible to detach it by force in the lengthwise direction L during removal.

According to another preferred mode of this embodiment, the difference between the dimension d_{W1} (Fig. 5(a): d_{W1L}, D_{W1R}) in the widthwise direction of the side wall section SW1 of one joint section 14a of the pair of joint sections 14a, 14b and the dimension d_{W1} (dW1_{L}, d_{W1R}) in the widthwise direction W of the side wall section SW1 of the other joint section 14b is within 40 µm. That is, the dimension d_{W1} of the side wall section SW1 in the widthwise direction W (the thickness of the cylinder in the widthwise direction) is approximately the same dimension for one joint section (for example, the joint section 14a) and the other joint section (for example, the joint section 14b) in the widthwise direction W. Therefore, when the wearer attempts to remove the disposable diaper 1, the left and right joint sections 14a, 14b can both be detached with approximately the same force. This allows the wearer to more easily detach the left and right joint sections 14a, 14b. In particular, since in the production method described above, the horns 42 and anvils 43 hold the plurality of cover sheets 5 stably for a prescribed period while fusing them, side wall sections SW1 with high shape symmetry can be stably formed.

According to another preferred mode of this embodiment, the difference between the inner side dimension (for example, Fig. 5(a): D_{W1L}) and the outer side dimension (for example, Fig. 5(a): d_{W1R}) in the widthwise direction W of each side wall section SW1 of the pair of joint sections 14a, 14b is within 40 µm. That is, the inner side dimension (for example, the cylinder thickness d_{W1L}) and the outer side dimension (for example, the cylinder thickness d_{W1R}) in the widthwise direction W are approximately the same dimension. At one side wall section SW1, therefore, the force necessary to detach the inner side section and the force necessary to detach the outer side section in the widthwise direction W are approximately the same. Therefore when the wearer attempts to remove the disposable diaper 1, it is possible to prevent the direction of force detaching the abdomen side section 11 and dorsal side section 13 from being in a direction diagonal to the lengthwise direction L or in the widthwise direction W, and therefore tearing of the abdomen side section 11 or dorsal side section 13 in a direction diagonal to the lengthwise direction L or in the widthwise direction W can be minimized.

According to a preferred mode of this embodiment, the fused section MC1 is located in the side wall section SW1 closer to the abdomen side section 11 than the dorsal side section 13 in the thickness direction T. Thus, when a wearer bows down to detach the abdomen side section 11 and dorsal side section 13 of their own disposable diaper 1, the fused sections MC1 are more easily visible, and therefore the wearer can pinch the area near the fused joint sections 30a with the fingers in a more reliable manner while detaching them. This will allow the abdomen side section 11 and dorsal side section 13 of the joint section 14a to be more easily detached.

According to another preferred mode of this embodiment, in each of the pair of joint sections 14a, 14b, the edge 11ae or 11be in the lengthwise direction L of the edge section 11a or 11b on the abdomen side section 11, and the edge 13ae or 13be in the lengthwise direction L of the edge section 13a or 13b on the dorsal side section 13, deviate from each other by Δc in the lengthwise direction L (preferably ±0.5 to 10 mm). Therefore when the wearer attempts to remove the disposable diaper 1, the wearer can more easily pinch the edge section 11a or 11b of the abdomen side section 11 with one finger, while pinching the edge section 13a or 13b of the dorsal side section 13 with the other finger. This will allow the abdomen side section 11 and dorsal side section 13 of the joint sections 14a, 14b to be more easily detached. If the absolute value of Δc is smaller than 0.5 mm it will be difficult to pinch the edge sections 11a, 11b with the finger, and if it is larger than 10 mm, the edge sections 11a, 11b may catch onto other objects and suffer damage.

### (Second embodiment)

In the first embodiment, each of the pair of joint sections 14a, 14b has the plurality of fused joint sections 30a, 30b, 30c disposed in a row along the lengthwise direction L with intervals between them. That is, the pressure welded section row 30L is a single row across the entire region in the lengthwise direction L. The plurality of fused joint sections 30a, 30b, 30c also have the same shape. For this embodiment, however, the pressure welded section row 32L in the center region 22 and the pressure welded section row 33L in the leg side edge region 23 are different from the pressure welded section row 31L in the waist side edge region 21, within the pressure welded section row 30L. This primary difference will now be explained in detail.

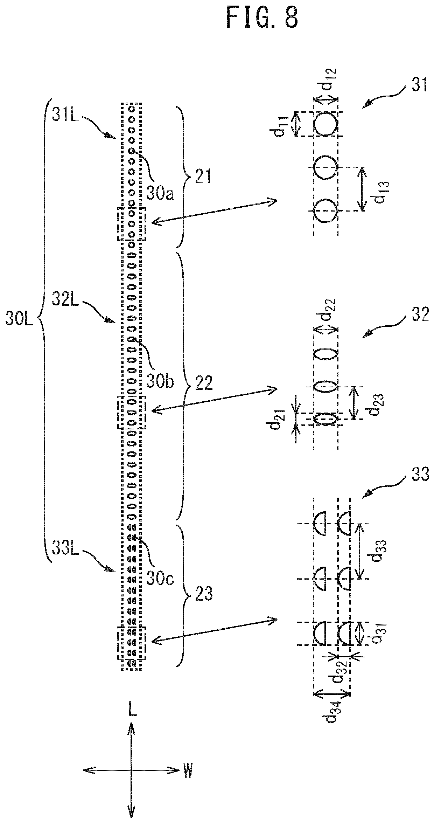

Fig. 8 is a schematic view for explaining another example of the construction of the joint section of Fig. 3. Fig. 8 is a schematic view of joint section 14a as seen from the dorsal side section 13 in the thickness direction T, showing an example of the arrangement of the fused joint sections 30. For this embodiment, the pressure welded section row 30L has one pressure welded section row 31L arranged along the lengthwise direction L in the waist side edge region 21, one pressure welded section row 32L arranged along the lengthwise direction L in the center region 22, and two pressure welded section rows 33L aligned in the widthwise direction W, arranged along the lengthwise direction L in the leg side edge region 23. For this embodiment, the fused joint sections 30a, 30b and 30c of the respective pressure welded section rows 31L, 32L and 33L have mutually different planar shapes. In other words, when the fused joint sections 30a, 30b and 30c are formed by an ultrasonic seal method, different planar shapes 31, 32, 33 are used for the tip sections of the ultrasonic horns for the fused joint sections 30a, 30b and 30c.

The planar shape 32 of the tip section of the ultrasonic horn used to form the fused joint section 30b of the pressure welded section row 32L are long elliptical in the widthwise direction W, and having: dimension d₂₁ in the lengthwise direction L < dimension d₂₂ in the widthwise direction W. The sizes of d₂₁ and d₂₂ are not particularly restricted, and may be 0.5 mm to 5 mm, for example. The distance d₂₃ between adjacent fused joint sections 30b in the lengthwise direction L (the distance between the long diameters of the ellipses) is not particularly restricted, and it may be 1 mm to 30 mm.

If d₂₂ is less than 0.5 mm, force applied from the ends of the patterns of fused sections during tearing will be applied over the entirety, instead of gradually, at the fused joint sections that are formed, although this will depend on the length of d₂₁, and therefore detachment will tend to take place more easily when the article is worn (by force along the widthwise direction W). If d₂₂ is greater than 5 mm, then force will be applied on the surface of the side wall section that has a large thickness in the lengthwise direction L when detachment takes place at the fused joint sections that are formed, and therefore detachment may be more difficult during removal. If d₂₁ is less than 0.5 mm, then the absolute amount of side wall section in the pattern of a single fused section with respect to the tearing surface will be smaller at the fused joint sections that are formed, although this will depend on the length of d₂₂, and therefore detachment will take place more easily when the article is worn. If d₂₁ is greater than 5 mm, then more of the side wall sections will be present in the tearing surfaces at the fused joint sections that are formed, and therefore detachment (by force along the lengthwise direction L) may be more difficult during removal. If the distance d₂₃ is less than 1 mm, more fused joint sections will be present on the tearing surface at the fused joint sections that are formed, and therefore it may be difficult to detach them (by force along the lengthwise direction) during removal, while if it is greater than 30 mm, conversely, fewer fused joint sections will be present at the fused joint sections that are formed, potentially making detachment more likely to occur when the article is worn.

The planar shape 33 of the tip section of the ultrasonic horn used to form the fused joint section 30c in the pressure welded section row 33L is a shape that is a semicircular with a chord in the widthwise direction W, and with the chord section of the semicircle slightly elongated in the widthwise direction W (semicircular + rectangular), two being aligned in an adjacent manner in the widthwise direction W. The dimension d₃₁ in the lengthwise direction L, the dimension d₃₂ in the widthwise direction W and the dimension d₃₄ between both ends of the tip sections aligned in the widthwise direction W are not particularly restricted, and may be 0.5 mm to 5 mm, for example. The distance d₃₃ between adjacent fused joint sections 30c in the lengthwise direction L (the distance between the centers of the (semi)circles) is not particularly restricted, and it may be 1 mm to 30 mm. The dimension d₃₄ between the outer edge sections of two adjacent fused joint sections 30c in the widthwise direction W is d₃₂ × 2 + α, where α is less than d₃₂ (>0).

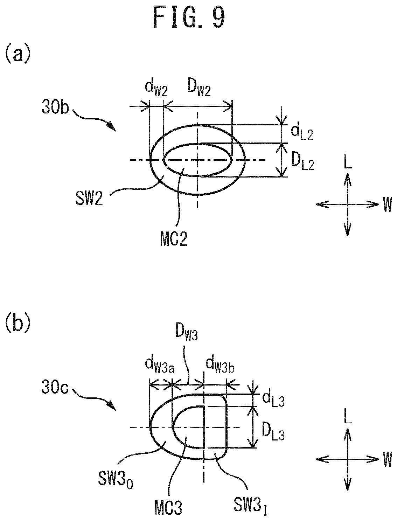

Fig. 9 is a plan view showing an example of the construction of a fused joint section 30 of the joint section 14a. Here, Fig. 9(a) shows the fused joint section 30b, and Fig. 9(b) shows the fused joint section 30c. Each of the fused joint sections 30b, 30c include fused sections MC2, MC3 and their respective side wall sections SW2, SW3.

The planar shape of the fused section MC3 of the fused joint section 30c is a shape that is semicircular with a chord in the widthwise direction W, and with the chord section of the semicircle slightly elongated in the widthwise direction W (semicircular + rectangular), two being aligned in an adjacent manner in the widthwise direction W. The dimension d_{L3} of the side wall sections SW3_{I}, SW3_{O} in the lengthwise direction L is smaller than the dimensions d_{W3a}, d_{w3b} in the widthwise direction W. This planar shape can generally be considered to be a shape that is half of the circle of the fused section MC1, aligned in the same direction. In this case, the fused joint section 30c, similar to the fused joint section 30a of the pressure welded section row 31L, can exhibit an effect of absorbing force in the widthwise direction W to prevent detachment while the article is being worn, while facilitating detachment by force in the lengthwise direction L during removal. In this case, d_{L3} is preferably 50 µm to 200 µm, similar to d_{L1}, while d_{W3a} and d_{w3b} are preferably 100 µm to 400 µm, similar to d_{W1}.

In addition to this, the fused joint section 30c has the straight linear chord section oriented toward the inner side in the widthwise direction W, in the plan view of the side wall section SW3_{I}. Therefore, even if force is applied from the inner side in the widthwise direction W when the article is worn, the force is absorbed over the wide flat surface of the chord section, thus making tearing of the side wall section SW3I less likely to occur compared to the side wall section SW1. In addition, the fused joint section 30c has two such side wall sections SW3 aligned along the widthwise direction W. Therefore, the fused joint section 30c can increase the resistance to detachment (resistance to tearing) in the widthwise direction W, compared to a single circular fused joint section 30a. In other words, it can increase the resistance against force from the inner side in the widthwise direction W when the article is worn.

Since the fused joint sections 30c of the pressure welded section row 33L are formed as two rows, force pulling apart the abdomen side section 11 and dorsal side section 13 in the lengthwise direction L can potentially be diffused between the two rows when the disposable diaper 1 is removed. Therefore, the two adjacent fused joint sections 30c in the widthwise direction W are placed in proximity in the widthwise direction W, and the dimension d₃₄ between their outer edge sections is calculated as d₃₂ × 2 + α (0 < α < d₃₂), thus allowing the abdomen side section 11 and dorsal side section 13 to be easily pulled apart during removal, essentially as a single row. For detachment from the waist opening WO side, however, since it is possible to detach the fused joint sections 30c of the leg side edge region 23 relatively easily with the momentum of detachment, the dimension d₃₄ between the outer edge sections does not necessarily need to be d₃₂ × 2 + α.

In the fused joint section 30b, the planar shape of the fused section MC2 is a long ellipsoid shape in the widthwise direction W, or in other words, the dimension D_{L2} of the fused section MC2 in the lengthwise direction L is smaller than the dimension D_{W2} in the widthwise direction W. In this case, the dimension d_{L2} of the side wall section SW2 in the lengthwise direction L may be made larger than the dimension d_{W2} in the widthwise direction W, and for this embodiment, the dimension d_{L2} in the lengthwise direction L may also be made larger than the dimensions d_{L1}, d_{L3} of the side wall sections SW1, SW3 in the lengthwise direction L (d_{L2} > d_{L1}, d_{L3}), and smaller than the dimensions d_{W1}, d_{W3a}, d_{w3b} of the side wall sections SW1, SW3 in the widthwise direction W (d_{W1}, d_{W3a}, d_{w3b} > d_{L2}). However, d_{L2} may also be about the same as or smaller than d_{w3b}. In this case, d_{L2} is preferably 70 µm to 250 µm.

The fused joint section 30b will then have lower ease of detachment in the lengthwise direction L than the fused joint sections 30a, 30c. Therefore, it is potentially possible that detachment will be more difficult with force in the lengthwise direction L during removal, than at the fused joint sections 30a, 30c. However, the fused joint sections 30a in the waist side edge region 21 (when detaching from the waist opening WO side) or the fused joint sections 30c in the leg side edge region 23 (when detaching from the leg opening LO side) exhibit sufficient effects for the function of facilitating detachment at the start of detachment (the start of tearing) during removal. When detachment begins (tearing begins), therefore, it is possible to detach the fused joint sections 30b in the center region 22 relatively easily with the momentum of detachment. Thus, as long as either or both the fused joint sections 30a in the waist side edge region 21 and the fused joint sections 30c in the leg side edge region 23 exhibit their function, it is not absolutely necessary for the function of each individual fused joint section 30b to be exhibited. The fused joint sections 30b can instead stop detachment (at the center region 22), when unwanted detachment has occurred at the fused joint sections 30a or the fused joint sections 30c when the article is worn. This can help prevent the disposable diaper 1 from becoming unusable due to damage at the joint sections 14a, 14b. From this viewpoint, d_{L2} > d_{L1}, d_{L3}, and also d_{W1}, d_{W3a}, d_{w3b} > d_{L2}, so that force of detachment of the abdomen side section 11 and dorsal side section 13 in the lengthwise direction L does not excessively increase during removal of the disposable diaper 1.

The dimension d_{W2} of the side wall section SW2 in the widthwise direction W is smaller than the dimension d_{L2} in the lengthwise direction L, and therefore smaller than the dimensions d_{W1}, d_{W3a}, d_{w3b} of the side wall sections SW1, SW3 in the widthwise direction W. Consequently, when the individual fused joint sections 30b are compared with the fused joint sections 30a, 30c, the former are less difficult to detach with force from the widthwise direction W when the article is being worn. Thus, they may also potentially be more easily detached by force in the widthwise direction W when the article is being worn. In terms of difficulty of detachment when the article is being worn, however, this is not a problem since the center region 22 is designed longer in the lengthwise direction L, and more of the fused joint sections 30b can diffuse and absorb force in the widthwise direction W when the article is being worn. Furthermore, it is also not a problem because the fused joint sections 30a in the waist side edge region 21 and the fused joint sections 30c in the leg side edge region 23 are more resistant to force in the widthwise direction W, and the force is diffused and absorbed over the entirety of the joint sections 14a, 14b. In this case, d_{W2} is preferably 60 µm to 200 µm.

Moreover, as mentioned above, it is not necessary for all of the fused joint sections among the joint section 14a to have the construction of the fused joint section 30a or fused joint sections 30c described above. It is sufficient if the section in the lengthwise direction L of the joint section 14a within a range of at least 20% from the edge section, which is the section on the edge section side where detachment starts (tearing starts), has the construction described above, the aforementioned percent being preferably at least 40% and more preferably at least 60%.

For this embodiment, the shapes of the fused sections for each of the pressure welded section rows 31L, 32L, 33L, i.e. the shapes of the tip sections of the ultrasonic horns, are preferably the same within each row. However, this embodiment is not limited to that example, and the shapes of the fused sections, i.e. the shapes of the tip sections of the ultrasonic horns in each pressure welded section row may differ either partially or totally. Moreover, the plurality of fused joint sections in each of the pressure welded section rows 31L, 32L, 33L may all be in a single row in the lengthwise direction L, or some or all of them may be in two, three or more rows. When each of the pressure welded section rows is a plurality of rows, each row is preferably in mutual proximity in the widthwise direction W, and from the viewpoint of easier detachment, preferably the edge sections of the facing side wall sections of adjacent fused joint sections are proximal to each other, at no greater than the dimensions of the fused joint sections in the widthwise direction W.

With this embodiment as well, the same function and effect can be exhibited as the function and effect of the first embodiment.

### EXAMPLES

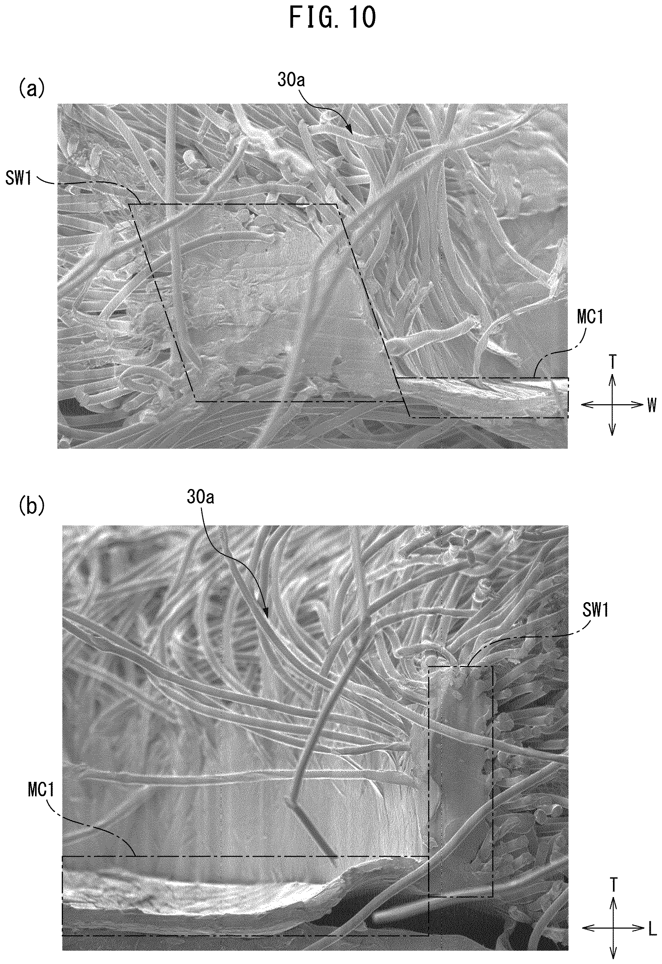

Examples, reference examples and comparative examples of the present invention will now be described for more concrete explanation of the present invention, with reference to Fig. 10 to Fig. 13, with the understanding that the present invention is not limited only to the examples.

### [Example 1]

In order to simulate a fused joint section 30a of a disposable diaper 1, an abdomen side section having one 15 g/m² SMS nonwoven fabric for a 25 mm × 25 mm cover sheet 5a and two 20 g/m² SB nonwoven fabrics for 25 mm × 25 mm cover sheets 5b layered, and a dorsal side section with the same construction, were overlaid, and one fused joint section 30a was formed by a joint section-forming apparatus 40 to prepare a sample. The planar shape 31 of the tip section of the horn 42 of the ultrasonic wave-generating means 41 used to form the fused joint section 30a was circular, the dimensions of the circle being d₁₁ = d₁₂ = 1.4 mm.

### [Comparative Example 1]

A sample was prepared in the same manner as Example 1. However, the planar shape of the tip section of the horn 42 of the ultrasonic wave-generating means 41 used to form the fused joint section was long elliptical in the widthwise direction W, the dimension of the ellipse in the lengthwise direction L being 1.0 mm and the dimension in the widthwise direction W being 2.0 mm.

Using the fused joint sections of the samples obtained in Example 1 and Comparative Example 1, the structures of cross-sections as seen from the widthwise direction W and cross-sections as seen from the lengthwise direction L were photographed with a scanning electron microscope (FlexSEM1000 by Hitachi, Ltd.) by the following photographing method and the dimensions of the fused sections and side wall sections of the samples were measured. The joining strength of the joint section of each sample was measured by the following measuring method. The tearing ease sensation for the joint section of each sample was also confirmed by touch.

### <Cross-sectional photography method>

i) A section including the fused joint section of the sample (approximately 2 mmcp for this example) is cut with scissors without directly holding the fused joint section with the hand, with one fused joint section at the center (this will hereunder be referred to as "cut strip"). During this time, the section including the fused joint section is cut into a quadrilateral shape (a 6 mm × 6 mm square for this example), so as to form a pressing area of at least a prescribed width (2 mm for this example) around the periphery of the fused joint section. For photographing of the cross-section of the fused joint section of the disposable diaper, a section without the elastic member is cut out to facilitate the measurement.
ii) The cut strip is placed on a stainless steel sheet (SUS304 2B), and locations in the pressing area on both sides in the direction perpendicular to the direction in which the fused joint section is to be cut (hereunder referred to as the "cutting direction") (locations 1 mm from each of both ends of the fused section of the fused joint section, for this example) are pressed with a pincette. A new cutter blade (cutter: SK Ltd-05 by Olfa Co., Ltd.) is used to cut the cut strip in half in the cutting direction, running through the center of the fused section.

Because of the risk that the fused joint section of the cut strip may collapse under the pressing pressure with the cutter blade, the cut strip is cut with the cutter blade in the following manner. First, the cutter blade is inserted into a section of the pressing area of the cut strip further outward than the fused joint section. However, the cutter blade is inserted at a tilt of 30 to 60° so that the blade is directed toward the fused joint section. The stainless steel sheet is then pressed with the cutter blade with a force of 15 N while the cutter is pulled in the cutting direction at a speed of 2 cm/sec, to cut the cut strip.
iii) The sample is then allowed to stand for 30 minutes so that the thickness of the cut section is restored to the thickness before cutting with the cutter blade.
iv) The cut section is fixed to the jig of a scanning electron microscope (SEM) using a pincette, and a SEM photograph is taken at the prescribed magnification (for example, 100x).

In this method of taking the cross-sectional photograph, the thickness of the sample or cut strip is determined by taking and measuring different locations of the same sample by SEM.

### <Joining strength measurement method>

The strength for detaching the abdomen side section and dorsal side section in the samples of Example 1 and Comparative Example 1, i.e. the joining strength, was measured by the following test method.
(i) The edge sections of the abdomen side section and dorsal side section of the sample in the lengthwise direction are clamped by the chuck of a tensile tester (chuck distance: 10 mm).
(ii) The abdomen side section and dorsal side section of the sample are pulled by the tensile tester, with a pull rate of 100 mm/min, to peel them at 180°, and the load value is measured. The tearing point at the fused joint section during this time is defined as 0 mm, and pulling is continued until the load is approximately zero.
(iii) The maximum of the measured load value is recorded as the joining strength.

Fig. 10 indicates examples of SEM photographs showing cross-sections of the fused joint section of Example 1. Fig. 10(a) is a cross-sectional photograph as seen from the lengthwise direction L, and Fig. 10(b) is a cross-sectional photograph as seen from the widthwise direction W. Based on these photographs, in Example 1, a discoid fused section MC1 was formed with D_{L1} ≈ D_{W1} ≈ 1.4 mm, D_{T1} ≈ 30 µm, and a slightly distorted cylindrical side wall section SW1 was formed with d_{L1} ≈ 100 µm, d_{W1} ≈ 160 µm, d_{T1} ≈ 180 µm.

While not shown here, the SEM photograph of the cross-section of the fused joint section of Comparative Example 1 showed that in Comparative Example 1, an elliptical discoid fused section had been formed with dimension in the lengthwise direction L ≈ 1.0 mm, dimension in the widthwise direction W ≈ 2.0 mm and dimension in the thickness direction T ≈ 30 µm, while a slightly distorted cylindrical side wall section had been formed with dimension in the lengthwise direction L ≈ 130 µm, dimension in the widthwise direction W ≈ 110 µm and dimension in the thickness direction T ≈ 160 µm.

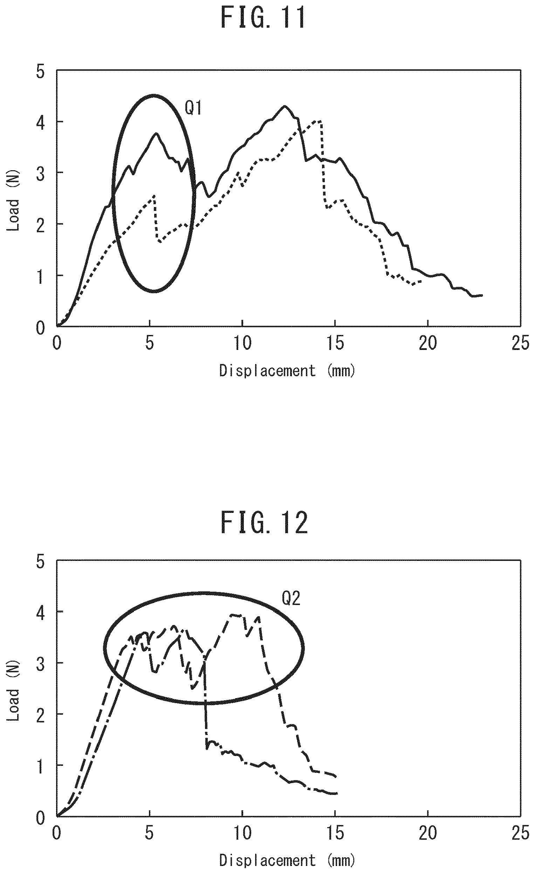

Fig. 11 to Fig. 13 are graphs showing the relationship between shape and strength of the fused joint section. The vertical axis represents load (N), and the horizontal axis represents displacement (mm).

Fig. 11 shows the load in the widthwise direction W for Example 1 and Comparative Example 1, the solid line representing Example 1 and the dotted line representing Comparative Example 1. As indicated by Q1 in the graph, in the widthwise direction W, the load in Example 1 (circular) was greater than the load in Comparative Example 1 (long ellipse in widthwise direction W). This suggests that d_{W1} in Example 1 was larger than the dimension in the widthwise direction W in Comparative Example 1. In other words, it may be concluded that Example 1 is less likely to detach by force in the widthwise direction W when worn, and thus has higher resistance.

Fig. 12 shows the load in the lengthwise direction L for Example 1 and Comparative Example 1, the dash-dot line representing Example 1 and the broken line representing Comparative Example 1. As indicated by Q2 in the graph, in the lengthwise direction L, the load in Comparative Example 1 (long ellipse in widthwise direction W) was greater than the load in Example 1 (circular). This suggests that the dimension in the lengthwise direction L in Comparative Example 1 was larger than d_{L1} in Example 1. In other words, it may be concluded that Example 1 is more likely to detach by force in the lengthwise direction L during removal, and thus has lower resistance.

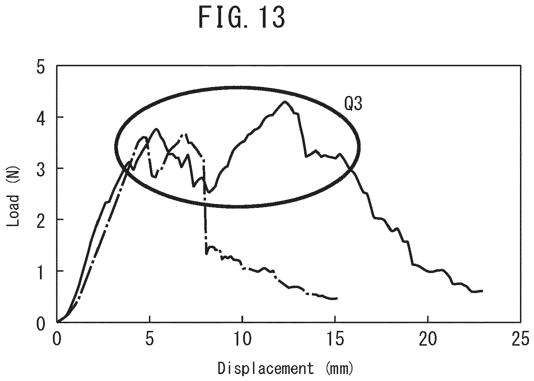

Fig. 13 shows the loads in the lengthwise direction L and widthwise direction W for Example 1, the solid line representing the load in the widthwise direction W and the dash-dot line representing the load in the lengthwise direction L. As indicated by Q3 in the graph, in Example 1, the load in the lengthwise direction L was smaller than the load in the widthwise direction W. This suggests that d_{L1} in Example 1 was less than d_{L2}. In other words, it may be concluded that in Example 1, detachment is more likely by force in the lengthwise direction L during removal, while detachment is less likely by force in the widthwise direction W when worn.

Also, with the sample of Example 1, the joining strength when detaching the fused joint section 30a in the lengthwise direction L was 3.5 N. The joining strength when detaching the fused joint section 30a in the widthwise direction W was 4.5 N. With the sample of Comparative Example 1, the joining strength when detaching the fused joint section in the lengthwise direction L was 4.0 N. The joining strength when detaching the fused joint section in the widthwise direction W was also 4.0 N.

These results confirmed that the joining strength at the fused joint section, i.e. the ease of detachment, varied depending on the thickness of the side wall section cylinder (for example, d_{L1}, d_{W1}), with a larger thickness having higher joining strength and a smaller thickness having lower joining strength, and that the sample of Example 1 had anisotropy of the ease of detachment due to the thickness of the side wall section cylinder at the fused joint section.

The tearing ease sensation for the joint section of each sample was also confirmed by touch. Specifically, the tearing ease sensation when tearing Example 1 and Comparative Example 1 in the lengthwise direction L was evaluated on a 5-level scale. Five collaborators were requested to perform the evaluation with "5" as the greatest tearing ease, "3" as normal, and "1" as the greatest tearing difficulty. The results are shown in Table 1.

**[Table 1]**

| Collaborator | Example 1 | Comparative Example 1 |
|---|---|---|
| A | 4 | 3 |
| B | 4 | 2 |
| C | 5 | 3 |
| D | 4 | 3 |
| E | 4 | 2 |
| Average | 4.2 | 2.6 |

As shown in Table 1, the sample of Example 1 was felt to be easier to tear.

The disposable diaper of the present invention is not restricted to the embodiments described above and can incorporate appropriate combinations and modifications without departing from the scope of the object and subject matter of the present invention. In other words, the techniques described for each of the embodiments may also be applied to other embodiments so long as they are not mutually contradictory.

### REFERENCE SIGNS LIST

1 Disposable diaper
11 Abdomen side section
13 Dorsal side section
14a, 14b Joint section
30 Fused joint section
MC, MC1-MC3 Fused section
SW, SW1-SW3 Side wall section

## Claims

1. An absorbent article comprising an abdomen side section and a dorsal side section, at least one edge section of the abdomen side section and one edge section of the dorsal side section in a widthwise direction being joined together by a joint section while overlapping in a thickness direction along a lengthwise direction, wherein:
the joint section comprises a plurality of fused joint sections,
each of the plurality of fused joint sections includes:
a fused section where a plurality of sheets on the abdomen side section and a plurality of sheets on the dorsal side section are fused together in the thickness direction, and
a side wall section where the plurality of sheets on the abdomen side section and the plurality of sheets on the dorsal side section are fused together in the thickness direction in a manner extending from a perimeter edge of the fused section cylindrically in the thickness direction,
a location of the fused section in the thickness direction deviates from a border between the abdomen side section and the dorsal side section,
a dimension of the side wall section in the thickness direction is larger than a dimension of the fused section in the thickness direction, and
a dimension of the side wall section in the lengthwise direction is smaller than a dimension in the widthwise direction.

2. The absorbent article according to claim 1, wherein
the joint section has a pressure welded section row in which the plurality of fused joint sections are arranged along the lengthwise direction with intervals between them.

3. The absorbent article according to claim 2, wherein
the joint section has only a single pressure welded section row as the plurality of fused joint sections.

4. The absorbent article according to claim 2, wherein:
the joint section has, in the lengthwise direction, a waist side edge region including an edge section close to a waist opening, a leg side edge region including an edge section far from the waist opening, and a center region between the waist side edge region and the leg side edge region, and
at least the waist side edge region and center region have only a single pressure welded section row as the plurality of fused joint sections.

5. The absorbent article according to any one of claims 1 to 4, wherein
a maximum dimension in the lengthwise direction of the fused section is greater than or equal to a maximum dimension in the widthwise direction.

6. The absorbent article according to any one of claims 1 to 5, wherein
a dimension in the lengthwise direction of the side wall section is 50 µm to 200 µm, and
a dimension in the widthwise direction of the side wall section is 100 µm to 400 µm.

7. The absorbent article according to claim 6, wherein
a pair of joint sections including the joint section are joined together respectively with both edge sections in the widthwise direction of the abdomen side section and the dorsal side section overlapping in the thickness direction along the lengthwise direction,
both of the pair of joint sections respectively comprise the plurality of fused joint sections, and
a difference between a dimension in the widthwise direction of the side wall section at one joint section of the pair of joint sections and a dimension in the widthwise direction of the side wall sections at the other joint section is within 40 µm.

8. The absorbent article according to claim 6 or 7, wherein
a difference between an inner side dimension and an outer side dimension of the side wall section of each of the pair of joint sections in the widthwise direction is within 40 µm.

9. The absorbent article according to any one of claims 1 to 8, wherein
the fused section is located inside the side wall section further toward the abdomen side section than the dorsal side section in the thickness direction.

10. The absorbent article according to any one of claims 1 to 9, wherein
at the joint section, an edge in the lengthwise direction on the abdomen side section and an edge in the lengthwise direction on the dorsal side section are mutually deviating in the lengthwise direction.

## Patentansprüche

1. Absorbierender Artikel, der einen Bauchseitenabschnitt und einen Rückenseitenabschnitt umfasst, wobei mindestens ein Randabschnitt des Bauchseitenabschnitts und ein Randabschnitt des Rückenseitenabschnitts in einer Breitenrichtung miteinander durch einen Verbindungsabschnitt bei Überlappung in einer Dickenrichtung entlang einer Längsrichtung verbunden sind, wobei:
der Verbindungsabschnitt eine Vielzahl von fusionierten Verbindungsabschnitten umfasst,
jeder der Vielzahl von fusionierten Verbindungsabschnitten Folgendes einschließt:
einen fusionierten Abschnitt, wo eine Vielzahl von Lagen auf dem Bauchseitenabschnitt und eine Vielzahl von Lagen auf dem Rückenseitenabschnitt miteinander in der Dickenrichtung fusioniert sind, und
einen Seitenwandabschnitt, der sich, wo die Vielzahl von Lagen auf dem Bauchseitenabschnitt und die Vielzahl von Lagen auf dem Rückenseitenabschnitt miteinander in der Dickenrichtung fusioniert sind, auf eine Weise von einem Umfangsrand des fusionierten Abschnitts zylindrisch in der Dickenrichtung erstreckt,
eine Stelle des fusionierten Abschnitts in der Dickenrichtung von einer Grenze zwischen dem Bauchseitenabschnitt und den Rückenseitenabschnitt abweicht,
eine Abmessung des Seitenwandabschnitts in der Dickenrichtung größer ist als eine Abmessung des fusionierten Abschnitts in der Dickenrichtung und
eine Abmessung des Seitenwandabschnitts in der Längsrichtung kleiner ist als eine Abmessung in der Breitenrichtung.

2. Absorbierender Artikel nach Anspruch 1, wobei
der Verbindungsabschnitt eine pressgeschweißte Abschnittsreihe aufweist, in der die Vielzahl von fusionierten Verbindungsabschnitten entlang der Längsrichtung mit Abständen zwischen ihnen angeordnet sind.

3. Absorbierender Artikel nach Anspruch 2, wobei
der Verbindungsabschnitt nur eine einzige pressgeschweißte Abschnittsreihe als die Vielzahl von fusionierten Verbindungsabschnitten aufweist.

4. Absorbierender Artikel nach Anspruch 2, wobei:
der Verbindungsabschnitt in der Längsrichtung Folgendes aufweist: einen Taillenseitenrandbereich, der einen Randabschnitt nahe zu einer Taillenöffnung einschließt, einen Beinseitenrandbereich, der einen Randabschnitt entfernt von der Taillenöffnung einschließt, und einen mittleren Bereich zwischen dem Taillenseitenrandbereich und dem Beinseitenrandbereich, und
zumindest der Taillenseitenrandbereich und mittlere Bereich nur eine einzige pressgeschweißte Abschnittsreihe als die Vielzahl von fusionierten Verbindungsabschnitten aufweisen.

5. Absorbierender Artikel nach einem der Ansprüche 1 bis 4, wobei
eine maximale Abmessung in der Längsrichtung des fusionierten Abschnitts größer ist als oder gleich einer maximalen Abmessung in der Breitenrichtung.

6. Absorbierender Artikel nach einem der Ansprüche 1 bis 5, wobei eine Abmessung in der Längsrichtung des Seitenwandabschnitts 50 µm bis 200 µm beträgt und
eine Abmessung in der Breitenrichtung des Seitenwandabschnitts 100 µm bis 400 µm beträgt.

7. Absorbierender Artikel nach Anspruch 6, wobei
ein Paar von Verbindungsabschnitten, die den Verbindungsabschnitt einschließen, miteinander jeweils mit beiden Randabschnitten in der Breitenrichtung des Bauchseitenabschnitts und Rückenseitenabschnitts bei Überlappung in der Dickenrichtung entlang der Längsrichtung verbunden sind,
beide des Paars von Verbindungsabschnitten jeweils die Vielzahl von fusionierten Verbindungsabschnitten umfassen und
eine Differenz zwischen einer Abmessung in der Breitenrichtung des Seitenwandabschnitts an einem Verbindungsabschnitt des Paars von Verbindungsabschnitten und einer Abmessung in der Breitenrichtung der Seitenwandabschnitte an dem anderen Verbindungsabschnitt innerhalb von 40 µm liegt.

8. Absorbierender Artikel nach Anspruch 6 oder 7, wobei
eine Differenz zwischen einer Innenseitenabmessung und einer Außenseitenabmessung des Seitenwandabschnitts von jedem des Paars von Verbindungsabschnitten in der Breitenrichtung innerhalb von 40 µm liegt.

9. Absorbierender Artikel nach einem der Ansprüche 1 bis 8, wobei
sich der fusionierte Abschnitt innerhalb des Seitenwandabschnitts weiter in Richtung des Bauchseitenabschnitts als des Rückenseitenabschnitts in der Dickenrichtung befindet.

10. Absorbierender Artikel nach einem der Ansprüche 1 bis 9, wobei
an dem Verbindungsabschnitt ein Rand in der Längsrichtung auf dem Bauchseitenabschnitt und ein Rand in der Längsrichtung auf dem Rückenseitenabschnitt voneinander in der Längsrichtung abweichen.

## Revendications

1. Article absorbant comportant une section côté abdomen et une section côté dorsal, au moins une section formant bord de la section côté abdomen et une section formant bord de la section côté dorsal dans une direction allant dans le sens de la largeur étant assemblées ensemble par une section d'assemblage tout en se chevauchant dans une direction allant dans le sens de l'épaisseur le long d'une direction allant dans le sens de la longueur, dans lequel :
la section d'assemblage comporte une pluralité de sections d'assemblage thermocollées,
chacune de la pluralité de sections d'assemblage thermocollées comprend :
une section thermocollée où une pluralité de feuilles sur la section côté abdomen et une pluralité de feuilles sur la section côté dorsal sont thermocollées ensemble dans la direction allant dans le sens de l'épaisseur, et
une section formant paroi latérale où la pluralité de feuilles sur la section côté abdomen et la pluralité de feuilles sur la section côté dorsal sont thermocollées ensemble dans la direction allant dans le sens de l'épaisseur d'une manière s'étendant depuis un bord de périmètre de la section thermocollée de manière cylindrique dans la direction allant dans le sens de l'épaisseur,
un emplacement de la section thermocollée dans la direction allant dans le sens de l'épaisseur dévie par rapport à une limite entre la section côté abdomen et la section côté dorsal,
une dimension de la section formant paroi latérale dans la direction allant dans le sens de l'épaisseur est supérieure à une dimension de la section thermocollée dans la direction allant dans le sens de l'épaisseur, et
une dimension de la section formant paroi latérale dans la direction allant dans le sens de la longueur est inférieure à une dimension dans la direction allant dans le sens de la largeur.

2. Article absorbant selon la revendication 1, dans lequel
la section d'assemblage a une rangée formant section soudée par pression dans laquelle les sections de la pluralité de sections d'assemblage thermocollées sont agencées le long de la direction allant dans le sens de la longueur avec des intervalles entre elles.

3. Article absorbant selon la revendication 2, dans lequel
la section d'assemblage a uniquement une seule rangée formant section soudée par pression pour la pluralité de sections d'assemblage thermocollées.

4. Article absorbant selon la revendication 2, dans lequel :
la section d'assemblage a, dans la direction allant dans le sens de la longueur, une région formant bord côté taille comprenant une section formant bord proche d'une ouverture au niveau de la taille, une région formant bord côté jambe comprenant une section formant bord éloignée de l'ouverture au niveau de la taille, et une région centrale entre la région formant bord côté taille et la région formant bord côté jambe, et
au moins la région formant bord côté taille et la région centrale ont uniquement une seule rangée formant section soudée par pression pour la pluralité de sections d'assemblage thermocollées.

5. Article absorbant selon l'une quelconque des revendications 1 à 4, dans lequel
une dimension maximum dans la direction allant dans le sens de la longueur de la section thermocollée est supérieure ou égale à une dimension maximum dans la direction allant dans le sens de la largeur.

6. Article absorbant selon l'une quelconque des revendications 1 à 5, dans lequel
une dimension dans la direction allant dans le sens de la longueur de la section formant paroi latérale fait de 50 µm à 200 µm, et
une dimension dans la direction allant dans le sens de la largeur de la section formant paroi latérale fait de 100 µm à 400 µm.

7. Article absorbant selon la revendication 6, dans lequel
une paire de sections d'assemblage comprenant la section d'assemblage sont assemblées ensemble respectivement avec les deux sections formant bord dans la direction allant dans le sens de la largeur de la section côté abdomen et de la section côté dorsal se chevauchant dans la direction allant dans le sens de l'épaisseur le long de la direction allant dans le sens de la longueur,
les deux sections de la paire de sections d'assemblage comportent respectivement la pluralité de sections d'assemblage thermocollées, et
une différence entre une dimension dans la direction allant dans le sens de la largeur de la section formant paroi latérale au niveau d'une section d'assemblage de la paire de sections d'assemblage et une dimension dans la direction allant dans le sens de la largeur des sections formant paroi latérale au niveau de l'autre section d'assemblage fait 40 µm au plus.

8. Article absorbant selon la revendication 6 ou la revendication 7, dans lequel
une différence entre une dimension latérale intérieure et une dimension latérale extérieure de la section formant paroi latérale de chacune de la paire de sections d'assemblage dans la direction allant dans le sens de la largeur fait 40 µm au plus.

9. Article absorbant selon l'une quelconque des revendications 1 à 8, dans lequel
la section thermocollée est située à l'intérieur de la section formant paroi latérale plus vers la section côté abdomen que vers la section côté dorsal dans la direction allant dans le sens de l'épaisseur.

10. Article absorbant selon l'une quelconque des revendications 1 à 9, dans lequel
au niveau de la section d'assemblage, un bord dans la direction allant dans le sens de la longueur sur la section côté abdomen et un bord dans la direction allant dans le sens de la longueur sur la section côté dorsal dévient mutuellement dans la direction allant dans le sens de la longueur.
